(19) Europäisches Patentamt
European Patent Office
Office européen des brevets

(11) EP 4 022 308 B1

(12) FASCICULE DE BREVET EUROPEEN

(45) Date de publication et mention de la délivrance du brevet:
**13.08.2025 Bulletin 2025/33**

(21) Numéro de dépôt: **20764086.3**

(22) Date de dépôt: **31.08.2020**

(51) Classification Internationale des Brevets (IPC):
***G01N 33/543*** (2006.01) ***G01N 27/74*** (2006.01)

(52) Classification Coopérative des Brevets (CPC):
**G01N 33/54326; G01N 27/745**

(86) Numéro de dépôt international:
**PCT/EP2020/074233**

(87) Numéro de publication internationale:
**WO 2021/038107 (04.03.2021 Gazette 2021/09)**

# (54) KIT ET METHODE DE CAPTURE D’UNE MOLECULE AVEC DES MOYENS MAGNETIQUES

KIT UND MOLEKÜL ZUR ERFASSUNG EINES MOLEKÜLS MIT MAGNETISCHEN MITTELN

KIT AND MOLECULE FOR CAPTURING A MOLECULE WITH MAGNETIC MEANS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA TN**

(30) Priorité: **30.08.2019 FR 1909561**

(43) Date de publication de la demande:
**06.07.2022 Bulletin 2022/27**

(73) Titulaires:
- **Centre national de la recherche scientifique
75016 Paris (FR)**
- **Institut Polytechnique de Grenoble
38031 Grenoble Cedex 1 (FR)**

(72) Inventeurs:
- **BLAIRE, Guillaume
74300 Magland (FR)**
- **CUGAT, Orphée
38320 Poisat (FR)**
- **VIEILLE, Victor
73160 Vimines (FR)**

(74) Mandataire: **Hautier IP
20, rue de la Liberté
06000 Nice (FR)**

(56) Documents cités:
**WO-A1-2014/111187 US-B1- 6 479 302**

- **ANONYMOUS: "Remanence", WIKIPEDIA, 26 July 2019 (2019-07-26), XP093124192, Retrieved from the Internet <URL:https://en.wikipedia.org/w/index.php?title=Remanence&oldid=907967715> [retrieved on 20240126]**
- **COUDRON L ET AL: "Low-cost credit card-based microfluidic devices for magnetic bead immobilisation", MICROFLUIDICS AND NANOFLUIDICS, SPRINGER, BERLIN, DE, vol. 14, no. 1 - 2, 18 September 2012 (2012-09-18), pages 359 - 369, XP035166850, ISSN: 1613-4990, DOI: 10.1007/S10404-012-1057-9**

Il est rappelé que: Dans un délai de neuf mois à compter de la publication de la mention de la délivrance du brevet européen au Bulletin européen des brevets, toute personne peut faire opposition à ce brevet auprès de l’Office européen des brevets, conformément au règlement d’exécution. L’opposition n’est réputée formée qu’après le paiement de la taxe d’opposition. (Art. 99(1) Convention sur le brevet européen).



Processed by Luminess, 75001 PARIS (FR)

## Description

La présente invention concerne un kit de capture d'une molécule. L'invention concerne également une méthode de capture d'une molécule.

Le test ELISA (acronyme du terme anglo-saxon « Enzyme-Linked Immuno Sorbent Assay ») est utilisé couramment pour diagnostiquer de manière quantitative des marqueurs moléculaires (antigènes, anticorps ou autre) présents dans des fluides, des biopsies, des cultures ou tout autre échantillon.

Cette technique, qui est à l'heure actuelle la plus robuste et une des plus répandues des méthodes de diagnostic, possède cependant des inconvénients, à savoir sa complexité, l'utilisation d'automates coûteux et sa durée qui peut atteindre quelques heures.

Le test ELISA est une technique d'immunodosage en phase hétérogène, c'est-à-dire qu'elle nécessite un support solide (typiquement, une plaque de titration comprenant une pluralité de puits) auquel on fixe au préalable une molécule adaptée pour capturer la molécule à doser.

Une fois que la molécule d'intérêt a été capturée sur ledit support, un lavage permet de retirer le reste de l'échantillon et de procéder à l'étape de détection et de quantification de ladite molécule.

Par exemple, dans le cas du test ELISA dit « sandwich », qui permet de doser un antigène dans une solution, on recouvre la surface du support d'une quantité déterminée d'un anticorps dit de capture, ledit anticorps étant adapté pour se lier à l'antigène recherché.

Puis on applique sur le support la solution susceptible de contenir ledit antigène ; ledit antigène se lie alors à l'anticorps de capture situé sur la surface du support.

Ensuite, on effectue un lavage du support de sorte à retirer l'antigène non lié restant éventuellement dans la solution. On dépose alors sur le support une solution contenant un anticorps dit anticorps de détection couplé à un moyen de détection, qui est adapté pour se lier à l'antigène fixé sur le support. Ledit anticorps de détection peut être directement marqué et émettre un signal détectable, mais peut également être couplé à une enzyme qui catalysera un substrat entrainant l'émission d'un signal détectable.

On met en œuvre une nouvelle étape de lavage, de sorte à conserver sur le support l'antigène lié à l'anticorps de détection, ledit anticorps étant lui-même couplé à l'enzyme.

Enfin, pour la détection et la quantification de l'antigène, on dépose sur le support un substrat qui est converti par l'enzyme en un signal détectable (par exemple une couleur analysée spectroscopiquement, ou par émission de fluorescence) représentatif de la liaison entre l'antigène et l'anticorps de détection.

Ledit signal peut être observé à l'oeil nu ou au moyen d'un instrument, tel qu'un spectrophotomètre.

L'article de D. Issadore et al, Lab Chip, 2011, 11, 147 décrit un procédé de capture d'une molécule dans un échantillon en faisant circuler ledit échantillon dans un microcanal fluidique agencé au-dessous d'une matrice de polydiméthylsiloxane (PDMS) dans laquelle des grains magnétiques de NdFeB ont été immobilisés.

Le document WO2014111187 décrit un procédé de capture d'une molécule dans un échantillon, comprenant les étapes suivantes : - le mélange dudit échantillon avec des particules magnétiques, chacune desdites particules étant couplée avec un élément apte à se lier sélectivement à ladite molécule à capturer, de sorte à former au moins un complexe comprenant une particule magnétique, ledit élément et ladite molécule liée audit élément, et - l'immobilisation dudit au moins un complexe sur un support comprenant des microsources de champ magnétique ordonnées.

Ces microsources de champ magnétique ordonnées sont réparties au voisinage de la surface du support destinée à être en contact avec l'échantillon selon un motif déterminé et présentent en outre une orientation magnétique déterminée.

La méthode décrite dans ce document est intéressante. Toutefois, le support de capture est difficile à réaliser et à industrialiser, notamment en environnement propre, ce qui implique un coût élevé de fabrication.

L'invention a notamment pour objectif de pallier ces inconvénients de l'art antérieur.

Plus précisément, l'invention a pour objectif de proposer un kit et une méthode de capture d'une molécule contenue dans un échantillon grâce à des moyens magnétiques performants et présentant une emprise financière réduite et compatible avec des techniques de production à bas coût.

Ainsi, l'invention concerne un kit de capture d'une molécule contenue dans un échantillon comprenant :

a) des nanoparticules magnétiques présentant comme plus grande dimension une dimension inférieure à 1 μm, lesdites nanoparticules étant chacune couplée à au moins un élément de capture, ledit au moins un élément de capture se liant spécifiquement à ladite molécule, et

b) un support de capture desdites nanoparticules magnétiques comprenant ou étant constitué essentiellement d'au moins une couche magnétique, ladite couche magnétique comprenant une juxtaposition, possiblement répétée, d'au moins une première et une deuxième région, la première région comprenant des particules magnétiques polarisées dans une première direction, et la deuxième région comprenant des particules magnétiques non polarisées ou polarisées dans une deuxième direction différente à la première direction de polarisation des particules magnétiques de la première région, de sorte que ladite au moins une couche magnétique génère un champ magnétique présentant au moins une variation d'intensité d'au moins 0,1 mT à une distance d'au moins 1 μm de ladite au moins une couche magnétique, ladite au moins une variation d'intensité du champ magnétique définissant un maximum et un minimum de la norme de l'intensité dudit champ magnétique, de sorte à définir au niveau dudit maximum de la norme dudit champ magnétique une zone de capture des nanoparticules magnétiques sur le support de capture,

ladite au moins une couche magnétique étant une bande magnétique souple comprenant des matériaux composites magnétiques aléatoirement distribués, ou orientés selon un axe de préorientation, dans un polymère, la couche magnétique présentant une retentivité de 2000 à 30000 μm.10-4T, la retentivité étant égale au moment magnétique de la couche magnétique divisé par la surface de la couche magnétique.

Les inventeurs ont découvert contre toute attente qu'il était possible d'attirer des nanoparticules couplées à un élément de capture à l'aide d'une couche magnétique présentant des particules magnétiques de faibles propriétés magnétiques.

Les couches magnétiques telles qu'utilisées dans l'invention sont souples et correspondent notamment à des bandes magnétiques. Les couches magnétiques de l'invention sont composées de matériaux composites magnétiques, tels que des ferrites, aléatoirement distribués dans un polymère ou bien orientés selon un axe de préorientation. Les ferrites sont une céramique ferromagnétique obtenue par moulage à forte pression et à haute température (>1 000 °C) à partir d'oxyde de fer $Fe_2O_3XO$, où X peut être du manganèse, du zinc, du cobalt, du nickel, baryum, strontium, etc.

L'invention consiste ainsi à détourner l'usage de bandes magnétiques couramment utilisées pour du stockage robuste, difficile à désaimanter, d'informations (cassette audio et vidéo, cartes de crédit, badges, tickets de transport, etc.) afin de les appliquer à la capture de particules magnétiques nanométriques en solution.

Dans un souci de clarté pour la suite de la description, les particules magnétiques composant une couche magnétique utilisée dans l'invention seront dénommées « poudres » ou « grains magnétiques », de manière à les distinguer clairement des « nanoparticules magnétiques » couplées aux éléments de capture.

Les couches magnétiques utilisées dans l'invention sont « codées », c'est-à-dire qu'au moins une partie des grains magnétiques les constituants sont polarisés/aimantés. Pour la suite, les termes « polarisé » et « aimanté » sont pris comme synonymes et seront utilisés uniformément.

Ce codage (ou cette polarisation) n'est pas réalisé de manière aléatoire, mais est configuré pour faire apparaitre au moins une juxtaposition d'une première région comprenant des grains magnétiques polarisés dans une première direction, et d'une deuxième région comprenant des grains magnétiques non polarisés ou polarisés dans une deuxième direction différente à la première direction de polarisation des grains magnétiques, définissant ainsi au moins une jonction entre une première et une deuxième région. Chaque région (lorsqu'elle est polarisée) émet donc son propre champ magnétique, de sorte que la couche magnétique peut être modélisée comme une pluralité de sources de champs magnétiques.

La polarisation des grains magnétiques composant ladite au moins une couche magnétique est notamment réalisée avec une tête d'écriture bien connue dans le domaine du codage de bande magnétique. Typiquement, un champ magnétique local est appliqué sur une région d'une couche magnétique au moyen d'un électroaimant miniature.

Cette juxtaposition particulière des premières et deuxièmes régions de polarisation permet de créer des variations, à une distance d'au moins 1 μm de ladite au moins une couche magnétique, de l'intensité du champ magnétique généré, et donc de créer des maximums et des minimums de la norme de l'intensité du champ magnétique. La norme de l'intensité du champ magnétique correspond à la valeur absolue de l'intensité du champ magnétique (en Tesla). Dans l'invention, les termes « norme de l'intensité » et « norme » peuvent être utilisés l'un à la place l'autre. Les maximums de la norme de l'intensité du champ magnétique créent des zones qui attirent lesdites nanoparticules en suspension et dans lesquelles lesdites nanoparticules magnétiques minimisent leur énergie magnétique de sorte qu'elles sont appelées « minimums locaux » de l'énergie magnétique des nanoparticules ou « puits d'énergie ».

Ainsi, par projection orthogonale sur la surface de ladite couche magnétique, les maximums de la norme de l'intensité du champ magnétique vont définir des zones de capture des nanoparticules. Les zones de captures et les puits d'énergie coïncident donc au même endroit.

Ces zones de captures s'étendent sur une distance d'au maximum 35 μm depuis la projection orthogonale sur la surface de ladite couche magnétique du ou de chaque maximum de la norme de l'intensité du champ magnétique. Par « au maximum 35 μm », il est entendu 35 μm, 34 μm, 33 μm, 32 μm, 31 μm, 30 μm, 29 μm, 28 μm, 27 μm, 26 μm, 25 μm, 24 μm, 23 μm, 22 μm, 21 μm, 20 μm, 19 μm, 18 μm, 17 μm, 16 μm, 15 μm, 14 μm, 13 μm, 12 μm, 11 μm, 10 μm, 9 μm, 8 μm, 7 μm, 6 μm, 5 μm, 4 μm, 3 μm, 2 μm et 1 μm.

L'énergie magnétique d'une nanoparticule (E) est égale à l'opposé du produit scalaire de l'aimantation de la nanoparticule ($\vec{M}$) par le champ magnétique ($\vec{B}$) généré par ladite au moins une couche magnétique selon la formule suivante :

$$E = -\vec{M}.\vec{B}$$

où E est l'énergie magnétique de la nanoparticule (en Joule),

M est l'aimantation de la nanoparticule (en Ampère par mètre), et

B est l'intensité du champ magnétique (en Tesla).

L'aimantation dans le cas des matériaux ayant des propriétés magnétiques utilisés dans l'invention est une fonction strictement croissante de l'intensité du champ magnétique, de sorte que les minimums de l'énergie magnétique des nanoparticules correspondent aux maximums de la norme du champ magnétique, et donc aux zones de capture.

Lorsqu'une nanoparticule magnétique est aimantée par le seul champ magnétique généré par ladite au moins une couche magnétique, les zones de capture se situent au niveau des jonctions d'une première et d'une deuxième région.

Le champ magnétique généré par ladite au moins une couche magnétique présente des variations d'intensité d'au moins 0,1mT et au plus de 1T, avantageusement d'au moins 0,1mT et au plus de 500mT, plus avantageusement d'au moins 0,5mT et d'au plus 300mT, encore plus avantageusement d'au moins 1mT et d'au plus 200mT.

Ces variations d'intensité du champ magnétique permettent de générer un gradient de champ magnétique fort, c'est-à-dire un gradient de champ magnétique suffisant pour exercer une force de capture significative par rapport au mouvement brownien des nanoparticules. Ainsi, un tel gradient de champ magnétique est localisé. En outre ledit gradient pointe vers une zone de capture et présente une valeur d'au moins 10 $T.m^{-1}$ à une distance de 10 $\mu$m de ladite au moins une couche magnétique, avantageusement de 10 $1.m^{-1}$ à $10^5$ $T.m^{-1}$, encore plus avantageusement de 500 $1.m^{-1}$ à $5*10^3$ $1.m^{-1}$. De cette manière, les gradients de champ magnétique forts guident les nanoparticules en suspension vers la ou les zones de capture de ladite au moins une couche magnétique.

Lorsque les nanoparticules magnétiques sont capturées par ladite au moins une couche magnétique, elles se positionnent au niveau de la ou de chaque zone de capture. Ce positionnement particulier est très intéressant pour détecter et quantifier directement les molécules capturées, comme il sera décrit plus en détail ci-après.

L'intensité du champ magnétique peut être mesurée avec une technique magnéto-optique dite MOIF (*Magneto-Optical Imaging Film*).

La technique MOIF est basée sur l'effet Faraday. De manière générale, cette technique consiste à plonger dans le champ magnétique d'un objet dont on souhaite mesurer l'intensité, un film plan composé d'un matériau dont les propriétés optiques sont affectées de manière connue par les champs magnétiques. Typiquement on accole ledit film audit objet. Ledit film plan présente une largeur et une longueur au moins égale à celle de la zone de l'objet testé. Suite à cette première étape, on illumine ledit film plan par un faisceau de lumière d'amplitude et de polarisation connues, ce faisceau traversant ledit film plan. L'analyse de la polarisation et de l'amplitude du faisceau de lumière ayant traversé ledit film plan donne une mesure des composantes planaires du champ magnétique présent en son sein. Un exemple de mesure de l'intensité du champ magnétique d'un objet par la technique MOIF est donné par l'article Grechishkin et al., J. Appl. Phys. 120, 174502 (2016*).*

En pratique, on dépose un film plan mince, dont l'épaisseur est typiquement inférieure au micromètre, qui est composé d'un matériau magnéto-optique (par exemple un grenat à base de terres rares) sur un substrat amagnétique transparent (par exemple du verre, du quartz ou de la silice), puis on le recouvre d'une couche réfléchissante très fine, un miroir (par exemple en d'or, argent ou aluminium), dont l'épaisseur est inférieure à 100 nm). Ainsi, le film de composé matériau magnéto-optique est recouvert sur une de ses faces d'un substrat amagnétique transparent, et sur l'autre face par une couche réfléchissante. Cet ensemble est accolé à un objet émettant un champ magnétique, tel qu'un support de capture utilisé dans l'invention. Puis, on illumine le film de matériau magnéto-optique avec un faisceau de lumière polarisée qui traverse en premier la couche amagnétique transparente (dont les capacités optiques ne sont pas influencées par le champ magnétique de l'objet magnétique et donc qui n'a pas d'incidence sur la polarisation dudit faisceau), traverse ensuite le film de matériau-optique (dont le champ magnétique généré par l'objet magnétique affecte les propriétés optiques et qui a donc un incidence sur la polarisation du faisceau), est réfléchi par la couche réfléchissante, traverse à nouveau le film de matériau-optique (qui affecte à nouveau la polarisation du faisceau), puis de nouveau le verre (qui n'a pas d'incidence sur la polarisation du faisceau) avant de terminer dans l'analyseur de polarisation. L'angle de rotation de la polarisation du faisceau réfléchi par rapport au faisceau incident est proportionnel au champ magnétique, au coefficient de rotation Faraday du grenat, et à l'épaisseur du matériau magnéto-optique. Il est ainsi obtenu une distribution de l'intensité du champ magnétique généré par ladite au moins une couche magnétique grâce à la courbe de calibration qui représente la rotation par effet Faraday du faisceau lumineux en fonction de l'intensité du champ magnétique. Cette courbe est spécifique au matériau magnéto-optique utilisé.

Notamment, l'intensité du champ magnétique peut être mesurée par la technique MOIF grâce à un système MagView CMOS commercialisé par la société MATESY GmBH avec comme analyseur de polarisation un capteur de modèle C, utilisant un grenat de type DLGi5 comme film de matériau magnéto-optique dont la courbe de calibrage est représentée en figure 11. Dans cet instrument, le miroir est remplacé par un capteur CMOS la lumière n'a donc pas à être réfléchie

Les résultats peuvent être confirmés par simulation numérique et analytiques, par exemple par l'une des deux approches suivantes :

- Une approche par éléments finis réalisée à l'aide du logiciel de modélisation COMSOL Multiphysics® 5.0). Ce logiciel permet de réaliser des simulations numériques dans un environnement bidimensionnel d'épaisseur 10mm. En amont sont mesurées d'une part l'épaisseur et la largeur de ladite au moins une couche magnétique, typiquement de manière optique à l'aide d'une image en microscopie en champ clair, et d'autre part ses valeurs de rémanence ou de retentivité, voir plus loin pour plus de détail. Ces données sont renseignées dans le logiciel, et le champ magnétique généré par ladite au moins une couche magnétique est simulé en utilisant la boite à outil MFNC (Magnetic Field No Current) dans un régime stationnaire.
- Une approche dite semi-analytique. Cette dernière est basée sur l'approche développée par exemple dans l'article de Chigirinsky S. et al, Advanced Study Center Co. Ltd., 20 (2009), 85-91. Ici aussi, en amont sont mesurées d'une part l'épaisseur et la largeur de ladite au moins une couche magnétique, et d'autre part les valeurs de rémanence ou de retentivité de ladite au moins une couche magnétique. Ladite au moins une couche magnétique est décomposée en une somme d'élément ayant une aimantation homogène, puis une résolution analytique des équations donnant le champ magnétique de chaque élément est faite par exemple sous le logiciel Scilab® 6.02 (éditeur Scilab Enterprises). Le champ généré par chaque élément de ladite au moins une couche magnétique est additionné à celui généré par l'ensemble des autres éléments de ladite au moins une couche magnétique en chaque point de l'espace. Dans le cas où au moins une source supplémentaire de champ magnétique est également présente, voir plus loin pour plus de détail, le champ magnétique généré par ladite au moins une source supplémentaire est additionné en chaque point au champ total généré par ladite au moins une couche magnétique.

Selon un mode de réalisation de l'invention, la deuxième région comprend des grains magnétiques polarisés dans une deuxième direction différente déviée d'au moins 30° par rapport à la première direction de polarisation des grains magnétiques de la première région, avantageusement déviée de 30° à 180°. Par « de 30° à 180° », il est entendu dans l'invention 30°, 31°, 32°, 33°, 34°, 35°, 36°, 37°, 38°, 39°, 40°, 41°, 42°, 43°, 44°, 45°, 46°, 47°, 48°, 49°, 50°, 51°, 52°, 53°, 54°, 55°, 56°, 57°, 58°, 59°, 60°, 61°, 62°, 63°, 64°, 65°, 66°, 67°, 68°, 69°, 70°, 71°, 72°, 73°, 74°, 75°, 76°, 77°, 78°, 79°, 80°, 81°, 82°, 83°, 84°, 85°, 86°, 87°, 88°, 89°, 90°, 91°, 92°, 93°, 94°, 95°, 96°, 97°, 98°, 99°, 100°, 101°, 102°, 103°, 104°, 105°, 106°, 107°, 108°, 109°, 110°, 111°, 112°, 113°, 114°, 115°, 116°, 117°, 118°, 119°, 120°, 121°, 122°, 123°, 124°, 125°, 126°, 127°, 128°, 129°, 130°, 131°, 132°, 133°, 134°, 135°, 136°, 137°, 138°, 139°, 140°, 141°, 142°, 143°, 144°, 145°, 146°, 147°, 148°, 149°, 150°, 151°, 152°, 153°, 154°, 155°, 156°, 157°, 158°, 159°, 160°, 161°, 162°, 163°, 164°, 165°, 166°, 167°, 168°, 169°, 170°, 171°, 172°, 173°, 174°, 175°, 176°, 177°, 178°, 179° ou 180°.

Avantageusement, la deuxième région comprend des grains magnétiques polarisés dans une deuxième direction différente déviée d'au moins 60°, plus avantageusement déviée d'au moins 90°, encore plus avantageusement déviée d'au moins 120°, encore plus avantageusement déviée d'au moins 150°.

Selon un mode avantageux de réalisation de l'invention, la deuxième région comprend des grains magnétiques polarisés dans une deuxième direction opposée à la première direction de polarisation des grains magnétiques de la première région, soit une inversion de polarisation de 180°.

Selon un autre mode de réalisation, les grains magnétiques de la deuxième région ne sont pas polarisés. Une telle configuration permet également l'apparition d'une variation de l'intensité du champ magnétique et donc un maximum de la norme de l'intensité du champ magnétique généré par ladite au moins une couche magnétique.

Selon un mode de réalisation de l'invention, ladite au moins une première région et ladite au moins une deuxième région présentent les mêmes dimensions. Alternativement, elles présentent des dimensions différentes, notamment des largeurs et/ou des longueurs différentes.

Selon un mode de réalisation de l'invention, ladite au moins une première région et/ou de ladite au moins une deuxième région présente une largeur allant de 10 à 500 μm. Par « de 10 à 500 μm », il est entendu dans l'invention 10 μm, 20 μm, 30 μm, 40 μm, 50 μm, 60 μm, 70 μm, 80 μm, 90 μm, 100 μm, 110 μm, 120 μm, 130 μm, 140 μm, 150 μm, 160 μm, 170 μm, 180 μm, 190 μm, 200 μm, 210 μm, 220 μm, 230 μm, 240 μm, 250 μm, 260 μm, 270 μm, 280 μm, 290 μm, 300 μm, 310 μm, 320 μm, 330 μm, 340 μm, 350 μm, 360 μm, 370 μm, 380 μm, 390 μm, 400 μm, 410 μm, 420 μm, 430 μm, 440 μm, 450 μm, 460 μm, 470 μm, 480 μm, 490 μm, 500 μm.

Avantageusement, ladite au moins une première région et/ou de ladite au moins une deuxième région présente une largeur allant de avantageusement de 50 à 250 μm, plus avantageusement de 70 à 150 μm, encore plus avantageusement de 90 à 110 μm.

Selon un mode de réalisation, ladite au moins une première région et ladite au moins une deuxième région forment le même motif. Ce motif peut correspondre notamment à une bande. Alternativement, elles représentent des motifs différents.

Selon un mode de réalisation ladite au moins une couche magnétique est revêtue par un film protecteur d'une épaisseur inférieure à 1 μm. Un tel film permet avantageusement de protéger ladite au moins une couche magnétique, sans pour autant gêner ses capacités de capture/d'attraction du fait de son épaisseur très faible.

Ladite au moins une couche magnétique peut à elle seule constituer le support de capture en tant que tel Dans ce cas, cette dernière présente avantageusement une épaisseur d'au moins 5 μm et plus avantageusement de 10 à 20μm.

Ladite couche magnétique est avantageusement disposée sur un organe de soutien.

Selon un mode de réalisation de l'invention, le matériau utilisé pour l'organe de soutien est choisi parmi la liste suivante : du verre, du silicium, un polymère de matériau plastique, de la matière organique tel que du papier ou du bambou, du quartz, de l'or, un ruban adhésif, un alliage métallique non-magnétique tel que du dural ou du titane, ou une combinaison de ces matériaux.

Avantageusement, le polymère est choisi parmi la liste suivante : du polydiméthylsiloxane (PDMS), du polyméthacrylate de méthyle (PMMA), un polymère de cyclo-oléfines (COP), un copolymère de cyclo-oléfines (COC), du polycarbonate, du polyimide, du polychlorure de vinyle (PVC), du polyéthylène, du polypropylène, du silicone, du polyester, ou une combinaison de ces matériaux.

L'organe de soutien peut être une simple couche d'un matériau listé ci-dessus.

La couche magnétique disposée sur l'organe de soutien peut être tendue par exemple à l'aide de deux bobines d'enroulement à l'instar d'une cassette VHS.

Selon un mode de réalisation de l'invention, le support de capture comprend un réceptacle de capture configuré pour recevoir l'échantillon contenant la molécule à capturer et délimité par au moins une paroi comprenant ladite au moins une couche magnétique. Ce réceptacle de capture présente comme plus petite dimension une dimension de 20 μm à 1000 μm. Par de « de 20 μm à 1000 μm » il est entendu dans l'invention 20 μm, 40 μm, 60 μm, 80 μm, 100 μm, 120 μm, 140 μm, 160 μm, 180 μm, 200 μm, 220 μm, 240 μm, 260 μm, 280 μm, 300 μm, 320 μm, 340 μm, 360 μm, 380 μm, 400 μm, 420 μm, 440 μm, 460 μm, 480 μm, 500 μm, 520 μm, 540 μm, 560 μm, 580 μm, 600 μm, 620 μm, 640 μm, 660 μm, 680 μm, 700 μm, 720 μm, 740 μm, 760 μm, 780 μm, 800 μm, 820 μm, 840 μm, 860 μm, 880 μm, 900 μm, 920 μm, 940 μm, 960 μm, 980 μm et 1000 μm.

Selon un mode de réalisation de l'invention, le support de capture est choisi parmi une chambre, une chambre parallélépipédique, un cylindre droit creux, un puits, un puits sous la forme d'un cône droit, notamment d'un cône droit tronqué ou pyramidale tronqué, un canal microfluidique, une plaque de titration, un tube à essai et un microtube.

Dans le cas d'une chambre et d'une chambre parallélépipédique, ladite au moins une couche magnétique est agencée au niveau d'une, et si plusieurs couches magnétiques sont présentes au niveau d'au moins une, paroi de ladite chambre.

Dans le cas d'un cylindre droit creux, ladite au moins une couche magnétique est agencée au niveau de la paroi circonférentielle dudit cylindre.

Dans le cas d'un puits, ladite au moins une couche magnétique est agencée au niveau d'une, et si plusieurs couches magnétiques sont présentes au niveau d'au moins une, paroi de dudit puits. Avantageusement, ladite au moins une couche magnétique est agencée au niveau de la paroi formant le fond dudit puits.

Dans le cas d'un puits sous la forme d'un cône, notamment d'un cône droit tronqué ou pyramidale tronqué, ladite au moins une couche magnétique est agencée au niveau d'une, et si plusieurs couches magnétiques sont présentes au niveau d'au moins une, paroi dudit puits.

Dans le cas d'un canal microfluidique, ladite au moins une couche magnétique est agencée au niveau d'une, et si plusieurs couches magnétiques sont présentes au niveau d'au moins une, paroi dudit canal.

Dans le cas d'une plaque de titration comprenant une pluralité de puits, ladite au moins une couche magnétique est agencée au niveau d'une, et si plusieurs couches magnétiques sont présentes au niveau d'au moins une, paroi d'au moins un puits. Avantageusement, ladite au moins une couche magnétique est agencée au niveau de la paroi formant le fond dudit au moins un puits.

Dans le cas d'un tube à essai ou d'un microtube, ladite au moins une couche magnétique est agencée au niveau d'une, et si plusieurs couches magnétiques sont présentes au niveau d'au moins une, paroi dudit tube à essai ou microtube. Avantageusement, ladite au moins une couche magnétique est agencée au niveau de la paroi circonférentielle dudit tube à essai ou microtube.

Ladite au moins une couche magnétique peut être fixée au support. Avantageusement, ladite fixation est irréversible. Dans ce cas, cette fixation peut être réalisée par exemple par collage, laminage ou emboutissage. Alternativement, ladite fixation est réversible. Ainsi, ladite au moins une couche magnétique peut être fixée par un système crochet et boucles plus communément appelé système velcro, ou bien par une colle réversible telle qu'une colle d'origine animale.

Le support de capture peut comprendre une ou plusieurs couches magnétiques utilisées dans l'invention.

Selon un mode de réalisation, ladite au moins une couche magnétique est repliée sur elle-même, de sorte à ce qu'une partie de ladite couche magnétique est superposée à une autre partie.

Selon un mode de réalisation de l'invention, ledit support de capture comprend au moins deux couches magnétiques. Avantageusement lesdites couches magnétiques sont disposées sur un même plan.

Alternativement, lesdites couches magnétiques sont disposées sur des plans différents, de sorte que lesdites deux ou au moins deux des couches sont superposées l'une sur l'autre.

Selon un autre mode de réalisation de l'invention, le support de capture comprend au moins une paroi d'attraction desdites nanoparticules magnétiques, ladite paroi comprenant ladite au moins une des couches magnétiques. Avantageusement, le support de capture comprend plusieurs parois d'attraction présentant chacune au moins une couche magnétique et au moins une desdites parois est disposée sur un plan différent de l'autre ou des autres couches magnétiques, avantageusement au moins une desdites parois est superposée sur l'une ou au moins une des autres parois. Alternativement ou de manière complémentaire, au moins une desdites parois est disposée orthogonalement à l'autre ou à au moins une des autres parois.

L'utilisation des couches magnétiques dans l'invention pour capturer des particules magnétiques nanométriques est contre-intuitive dans la mesure où, de manière courante, la capture de ces nanoparticules est réalisée grâce à des couches magnétiques présentant de fortes propriétés magnétiques, comme par exemple des couches magnétiques constituées d'alliages à base de terres rares. En effet, pour capturer des nanoparticules, qui présentent de manière inhérente une aimantation faible, en raison de leur petit volume. Il est de coutume d'utiliser des couches magnétiques présentant de fortes propriétés magnétiques. Des exemples de telles couches magnétiques « fortes » à base de terres rares sont notamment décrits dans le document WO2014111187. Les couches magnétiques fortes présentent une rémanence de 0,7 T à 1,5 T et un champ coercitif de 600kA/m à 2400kA/$m^3$.

Ladite au moins une couche magnétique selon l'invention présente des valeurs 5 à 15 fois plus faibles de rémanence apparente et de retentivité. Le champ coercitif de ladite au moins une couche magnétique va de 10 à 400 kA/$m^3$.

Par « rémanence apparente » il est entendu la rémanence de ladite au moins une couche magnétique prise dans son ensemble, et non de chacun des grains magnétiques la constituant.

Le champ coercitif d'un matériau ferromagnétique désigne l'intensité du champ magnétique qu'il est nécessaire d'appliquer, à un matériau ayant initialement atteint son aimantation à saturation, pour annuler l'aimantation du matériau.

La rémanence est une grandeur intensive du matériau, qui mesure l'induction ou la densité de flux magnétique qui persiste dans un matériau ferromagnétique après avoir été aimanté à l'aide d'un fort champ magnétique extérieur. La rémanence se mesure en Tesla (T). Un échantillon de matériau magnétique permanent, préalablement aimanté possède un moment magnétique proportionnel à son volume et à la rémanence du matériau. Un moment magnétique est une grandeur vectorielle qui permet de caractériser l'intensité d'une source magnétique. Le flux magnétique généré par cet échantillon est proportionnel à son moment. Ce flux magnétique peut être mesuré dans un magnétomètre à échantillon vibrant (VSM), ou à extraction, ou à SQUID. Typiquement, on applique à l'échantillon un champ magnétique suffisamment fort (typiquement 4 à 6 Tesla) suivant son axe d'aimantation préférentiel pour saturer son aimantation, puis on arrête ce champ magnétique dit « de saturation ». La mesure du flux généré par l'échantillon sous un champ magnétique nul (0 T) après saturation donne le moment magnétique rémanent de l'échantillon. On obtient alors la rémanence du matériau qui est égale au moment de l'échantillon divisé par le volume de l'échantillon.

Selon un mode de réalisation de l'invention, ladite au moins une couche magnétique présente une rémanence inférieure ou égale à 0,6 T, avantageusement une rémanence de 0,01 T à 0,6 T. Par « de 0,01 T à 0,6 T », il est entendu dans l'invention 0,01 T, 0,02 T, 0,03 T, 0,04 T, 0,05 T, 0,06 T, 0,07 T, 0,08 T, 0,09 T, 0,1 T, 0,11 T, 0,12 T, 0,13 T, 0,14 T, 0,15 T, 0,16 T, 0,17 T, 0,18 T, 0,19 T, 0,2 T, 0,21 T, 0,22 T, 0,23 T, 0,24 T, 0,25 T, 0,26 T, 0,27 T, 0,28 T, 0,29 T, 0,3 T, 0,31 T, 0,32 T, 0,33 T, 0,34 T, 0,35 T, 0,36 T, 0,37 T, 0,38 T, 0,39 T, 0,4 T, 0,41 T, 0,42 T, 0,43 T, 0,44 T, 0,45 T, 0,46 T, 0,47 T, 0,48 T, 0,49 T, 0,5 T, 0,51 T, 0,52 T, 0,53 T, 0,54 T, 0,55 T, 0,56 T, 0,57 T, 0,58 T, 0,59 T, 0,6 T.

Plus avantageusement, ladite au moins une couche magnétique présente une rémanence de 0,02 T à 0,5 T, encore plus avantageusement de 0,05 T à 0,2 T.

Lorsque l'épaisseur de ladite couche magnétique ou l'ensemble des couches magnétique est trop fin, c'est-à-dire lorsqu'elle ou il présente une largeur et/ou une longueur très supérieure, au moins 10 fois supérieure, à son épaisseur, il devient alors difficile de déterminer le volume du matériau magnétique et donc de calculer sa rémanence. C'est notamment le cas des bandes magnétiques disponibles dans le commerce pour lesquelles une couche magnétique fine repose sur une couche de substrat et, où qui plus est la limite entre ces deux couches est souvent difficile à évaluer à cause des processus de fabrication industriels. Dans ce cas, on mesure plutôt la rétentivité de la couche magnétique ou de l'ensemble de couches magnétique. La retentivité est égale au moment magnétique de l'échantillon divisé par la surface de l'échantillon (et non plus son volume). La retentivité est exprimée en unité de densité surfacique de flux magnétique, c'est-à-dire en μm.Gauss (1 μm.Gauss correspond à 1 μm.$10^{-4}$ T). Typiquement, un échantillon de 2mm x 2mm (taille caractéristique pour entrer dans un magnétomètre de laboratoire) est découpé au sein du support de capture à tester. Sa surface exacte est mesurée au microscope optique. La marche à suivre pour obtenir la retentivité du matériau magnétique est la même que pour la rémanence, c'est-à-dire qu'on va procéder à la saturation de son aimantation afin d'obtenir son moment magnétique.

Ainsi, l'invention concerne un kit tel que défini précédemment, dans lequel ladite au moins une couche magnétique présente une retentivité de 2000 à 30000 μm.Gauss (2000 à 30000 μm.$10^{-4}$ T). Par « 2000 à 30000 µm.Gauss », il est entendu dans l'invention 2000 µm.Gauss, 2500 µm.Gauss, 3000 µm.Gauss, 3500 µm.Gauss, 4000 µm.Gauss, 4500 µm.Gauss, 5000 µm.Gauss, 5500 µm.Gauss, 6000 µm.Gauss, 6500 µm.Gauss, 7000 µm.Gauss, 7500 µm.Gauss, 8000 µm.Gauss, 8500 µm.Gauss, 9000 µm.Gauss, 9500 µm.Gauss, 10000 µm.Gauss, 11000 µm.Gauss, 12000 µm.Gauss, 13000 µm.Gauss, 14000 µm.Gauss, 15000 µm.Gauss, 16000 µm.Gauss, 17000 µm.Gauss, 18000 µm.Gauss, 19000 µm.Gauss, 20000 µm.Gauss, 21000 µm.Gauss, 22000 µm.Gauss, 23000 µm.Gauss, 24000 µm.Gauss, 25000 µm.Gauss, 26000 µm.Gauss, 27000 µm.Gauss, 28000 µm.Gauss, 29000 µm.Gauss, 30000 µm.Gauss.

Selon un mode de réalisation de l'invention, ladite au moins une couche magnétique présente une retentivité de 5000 à 20000 µm.Gauss (5000 à 20000 $\mu m.10^{-4}$ T), avantageusement de 8000 à 14000 µm.Gauss, plus avantageusement de 9000 à 11000 µm.Gauss. Les nanoparticules magnétiques capturées présentent comme plus grande dimension une dimension inférieure à 1 µm.

De par leurs dimensions, les particules magnétiques employées présentent des propriétés superparamagnétiques.

Le terme « superparamagnétique » désigne la propriété de particules de matériau ferromagnétique ou ferrimagnétique de petites dimensions de changer aléatoirement de direction d'aimantation en l'absence d'un champ magnétique appliqué, sous l'effet de l'agitation thermique.

Le caractère « superparamagnétique » des particules magnétiques implique qu'en l'absence de champ magnétique d'excitation extérieur, les particules magnétiques n'ont aucun moment magnétique net, de sorte qu'elles ne s'attirent pas mutuellement, ce qui évite leur agglomération.

Par rapport à des microparticules couplées à des éléments de capture, les nanoparticules couplées aux éléments de capture présentent des performances beaucoup plus importantes en termes de capture des molécules, dues notamment à un plus fort coefficient de diffusion (multiplié par 10) et une concentration (nombre de billes au $m^3$) très largement augmenté (multiplié par $10^3$). Toutefois, la force magnétique de chacune d'elle est divisée par $10^3$. (chiffres dans le cas de taille réduite 1/10)

Selon un mode de réalisation de l'invention, les nanoparticules magnétiques présentent comme plus grande dimension une dimension allant de 50 nm à 500 nm, avantageusement de 50 nm à 250 nm, plus avantageusement de 100 à 250 nm, encore plus avantageusement de 150 à 200 nm.

Selon un mode de réalisation, les nanoparticules magnétiques comprennent de 10 à 90 % de fer, avantageusement de 30 à 80%, plus avantageusement de 50 à 70% de fer. Plus la quantité de fer que contiendra les nanoparticules sera important, plus leur aimantation en présence d'un champ magnétique supplémentaire sera grande et plus leur attraction par ladite au moins une couche magnétique sera forte. Ainsi plus la quantité de fer sera importante, moins il sera nécessaire d'augmenter leur aimantation pour qu'elles soient plus rapidement attirées, comme il sera vu plus loin.

Selon un mode de réalisation de l'invention, les nanoparticules sont encapsulées. Elles peuvent être notamment obtenues par copolymérisation d'oxyde de fer et de polystyrène. Cette encapsulation permet de limiter un relargage de fer des nanoparticules. En effet, un tel relargage perturbe la détection et la quantification de la molécule capturée.

Les nanoparticules magnétiques peuvent présenter toutes formes telles que parallélépipédique, torique, sphérique, etc. Les nanoparticules peuvent présenter une surface lisse ou irrégulière. Lorsqu'elles présentent une surface irrégulière, elles de forme dite « patatoïde ».

Avantageusement, les nanoparticules magnétiques sont sphériques et sont alors apparentées à des « billes ». Les particules pourront donc être désignées par le terme « billes » même si leur géométrie n'est pas une sphère parfaite.

De préférence, lesdites billes sont monodispersées, l'uniformité dimensionnelle des billes leur conférant des propriétés identiques et améliorant ainsi la diffusion des billes pour leur capture par ladite au moins une couche magnétique. Par « monodispersées », il est entendu que l'écart type de la moyenne du diamètre des billes est inférieur ou égal à 40 nm sur 200nm avantageusement 20nm sur 200nm.

Dans certains cas, les billes sont commercialisées sous une forme dispersée dans une matrice peu ou pas magnétique, telle qu'un polymère de matière plastique, de la silice ($SiO_2$), etc.

Les billes sont de préférence biocompatibles, c'est-à-dire qu'elles ont la capacité à ne pas interférer, ne pas dégrader, le milieu biologique dans lequel elles sont utilisées.

Pour permettre le couplage d'un élément de capture aux nanoparticules magnétiques, la surface de ces dernières est fonctionnalisée, notamment par des protéines A ou G de *Staphylococcus aureus* ou encore par un carbodiimide Dans le cas de l'utilisation des protéines A ou G, la liaison entre l'élément de capture et les nanoparticules ne sera pas covalente, au contraire de l'utilisation d'un carbodiimide.

Divers éléments de capture peuvent être couplés aux nanoparticules. Selon un mode de réalisation de l'invention, l'élément de capture est choisi parmi un anticorps, un fragment Fab, un fragment F(ab')2 ou un fragment Fv d'un anticorps, un antigène, une séquence d'acide nucléique, un organite correspondant notamment à une vésicule, une cellule, un aptamère ou une bactérie.

Un anticorps ou « immunoglobuline » est constituée de 4 chaînes d'acides aminés, où l'on distingue deux chaînes légères et deux dites lourdes. Chaque chaine lourde est liée par un pont disulfure à une chaine légère. En outre, l'extrémité d'une chaine lourde et celle de la chaine légère associée définissent ensemble un paratope grâce à des régions hypervariables. Un anticorps comprend ainsi deux paratopes permettant chacun la liaison avec un épitope d'un antigène. Les chaines lourdes sont reliées entre elles au niveau d'une région dite charnière.

Le fragment de l'anticorps correspondant à un des deux paratopes est appelé Fragment Fv, c'est le plus petit fragment d'un anticorps gardant les propriétés de reconnaissance d'un épitope.

Le fragment Fab correspond quant à lui à une chaine légère entière et l'extrémité de la chaine lourde liée à cette chaine légère. Un fragment Fab comprend ainsi un fragment Fv. Il y a deux fragments Fab pour un anticorps.

Le fragment F(ab')2 correspond à l'association des deux fragments Fab reliés entre eux par la région charnière des chaines lourdes.

Les fragments Fv, Fab et F(ab')2 présente la même affinité pour un antigène que l'anticorps complet.

Un acide nucléique est un polymère dont l'unité de base est le nucléotide. Un acide nucléique peut correspondre à de l'acide désoxyribonucléique (ADN) ou à de l'acide ribonucléique (ARN).

Un organite est un compartiment différencié contenu dans le cytoplasme des cellules eucaryotes et dans lequel sont réalisées des fonctions biologiques particulières. En particulier, parmi les organites, on retrouve le réticulum endoplasmique, l'appareil de Golgi, les mitochondries, les lysosomes ou encore les peroxysomes.

Une vésicule est un compartiment présent dans le cytoplasme d'une cellule et constitué par au moins une bicouche lipidique. Les vésicules circulent dans le cytosol et présentent diverses fonctions comme le stockage, le transport, ou encore la digestion des déchets cellulaires.

Une cellule est un compartiment constituant les êtres vivants, elle est limitée par une membrane et comprend d'une part de l'ADN, nécessaire à sa reproduction, et d'autre par des protéines, nécessaires à son fonctionnement.

Un aptamère est un oligonucléotide synthétique, le plus souvent un ARN qui est capable de fixer un ligand spécifique et parfois de catalyser une réaction chimique sur ce ligand1. Les aptamères sont en général des composés synthétiques, isolés *in vitro à* partir de banques combinatoires d'un grand nombre de composés de séquence aléatoire par une méthode de sélection itérative appelée « évolution systématique de ligands par enrichissement exponentiel » (SELEX). De plus amples détails sur la synthèse d'aptamères par la méthode SELEX peuvent être trouvés dans l'article « Aptamers and SELEX in Chemistry & Biology » (Chem Biol. 2014 Sep 18 ; 21(9) : pages 1055-8).

Une bactérie est un micro-organisme unicellulaire procaryote comprenant un unique compartiment cytoplasmique contenant l'ADN. Ainsi cet ADN, contrairement aux cellules eucaryotes, n'est pas isolé du cytoplasme par un noyau. Les bactéries se reproduisent en se divisant simplement en deux par scissiparité.

Le type d'élément de capture à utiliser sera facilement adapté par l'homme du métier en fonction du type de molécule à capturer.

L'invention concerne également un kit tel que défini précédemment comprenant en outre au moins une source de champ magnétique supplémentaire, ladite source supplémentaire étant externe à ladite au moins une couche magnétique.

Le champ magnétique généré par ladite au moins une source de champ magnétique supplémentaire va avoir plusieurs impacts sur les éléments du kit, qui permettent d'une part d'accélérer la capture des nanoparticules par le support de capture, et d'autre part d'obtenir des zones de capture de capture plus localisées, c'est-à-dire plus précise, moins large, typiquement s'étendent sur une distance inférieure à 15 $\mu$m depuis la projection orthogonale sur la surface de ladite couche magnétique.

D'une part, l'application du champ magnétique de ladite au moins une source de champ magnétique supplémentaire permet avantageusement d'augmenter l'aimantation des nanoparticules magnétiques et ainsi d'accélérer, voire de déclencher leur capture par ladite au moins une couche magnétique du support de capture.

D'autre part, la présence d'un champ magnétique supplémentaire de norme supérieure ou égale à celle du champ magnétique généré par ladite au moins une couche magnétique s'additionne au champ magnétique généré par ladite au moins une couche magnétique de sorte que, selon l'orientation du champ magnétique supplémentaire l'amplitude de certains puits d'énergie du champ magnétique total résultant est supérieure à celles des puits d'énergie du seul champ magnétique généré par ladite au moins une couche magnétique, ce qui participe également à l'accélération de la capture des nanoparticules.

Les zones de capture sont renforcées, à la surface du support, là où le champ magnétique généré au-dessus des jonctions entre premières et deuxièmes régions par ladite au moins une couche magnétique suit le même axe et le même sens que le champ magnétique supplémentaire. La norme de l'intensité du champ magnétique total s'en trouve augmentée significativement. Au contraire, au-dessus des premières et deuxièmes régions, la norme de la résultante du champ magnétique supplémentaire avec celui généré par ladite au moins une couche magnétique n'est pas augmentée d'autant car ils ne sont pas colinéaires. Les maximums de la norme de l'intensité du champ magnétique sont donc plus fortement augmentés que les minimums, l'amplitude de certains puits d'énergie est donc accentuée. Ceci, d'une part accélère la capture sur ces zones de capture, qui sont plus fortement attirées par ces puits d'énergie, et d'autre part diminue l'étendue des zones de capture, qui sont donc géographiquement mieux définies et plus précises.

Lorsque le champ magnétique généré par ladite au moins une source supplémentaire au niveau d'une jonction entre premières et deuxièmes régions, à la surface du support est supérieure au champ magnétique généré par ladite au moins une couche magnétique selon le même axe mais dans un sens opposé, les zones de capture sont supprimées. En effet, la norme de l'intensité du champ magnétique généré à ce niveau n'est plus un maximum, et l'énergie des nanoparticules n'y est plus minimisée.

Dans le cas particulier où la polarisation des premières et secondes régions est opposée et parallèle à la surface du support, avec le champ magnétique supplémentaire allant dans la même direction que le champ magnétique généré conjointement par lesdites premières et deuxièmes régions au niveau de leur jonction à la surface du support, une zone de capture sur deux est renforcée et une zone de capture sur deux est affaiblie voire annulée pour les raisons évoquées ci-dessus.

Lorsque le champ supplémentaire est supérieur ou égal au champ magnétique généré par la dite au moins une source de champ magnétique, la zone de capture se situe au niveau de l'endroit où le champ magnétique supplémentaire est de même orientation et de même sens que le champ généré par ladite au moins une bande magnétique. Les zones de capture peuvent alors être décalée par rapport à leur position initiale, comme détaillé plus loin.

L'aimantation d'un objet correspond à une grandeur vectorielle qui caractérise à l'échelle macroscopique le comportement magnétique dudit objet. Elle a comme origine le moment magnétique orbital et le moment magnétique de spin des électrons. Elle se mesure en ampères par mètre ou, parfois, en Tesla.

Avantageusement, ladite au moins une source de champ magnétique supplémentaire est choisie parmi un aimant permanent, une bobine, ou un électro-aimant.

Lorsque plusieurs sources de champ magnétiques sont présentes, elles peuvent être choisies parmi une combinaison d'au moins un aimant permanent, d'au moins une bobine et/ou d'au moins un électro-aimant. Notamment, elles peuvent être choisies parmi un assemblage d'aimants permanents, de bobines, d'électro-aimants et une combinaison d'assemblage de ces derniers.

Les sources de champ magnétique peuvent être disposées côte à côte selon un plan, notamment de manière linéaire, ou bien selon une forme tridimensionnelle. Les sources de champs magnétiques peuvent être disposées côte à côte. Par « côte à côte », il est entendu dans l'invention que ces dernières sont accolées ou bien espacées les unes des autres. Notamment, les sources de champ magnétique qui sont adjacentes présentent une inversion de polarisation.

Un aimant permanent est un objet fabriqué dans un matériau magnétique dur ayant acquis, artificiellement ou naturellement, durablement la propriété de générer un champ magnétique. La particularité d'un aimant permanent réside dans le fait que son champ magnétique une fois acquis est généré continuellement sans nécessité d'une action particulière. Par matériau magnétique dur, il est entendu un matériau dont l'aimantation rémanente et le champ coercitif sont grands, supérieur à 0,3 T et à 250 kA/m respectivement.

Le matériau magnétique dur peut être choisi parmi un aimant à base de terres rares, un alliages métal de transition de la série 3d (Fe, Co, Ni)-métal noble (Pt ou Pd comme élément majoritaire), un aimant ferrite et un aimant MnBi, MnAl, MnGa, FeGa, AlNiCo. Lorsque le matériau est un aimant à base de terres rares, il peut choisi parmi RFeB (où R consiste en Nd, Pr, Tb, Dy ou un mélange de plusieurs de ces éléments), SmCo ou RCo-Cu (structure cristallographique de type 1/5), SmCoCuFe (structures cristallographiques de type 1/7 ou 2/17), RFeN (où R consiste essentiellement en Sm).

Pour la suite de la description, le terme « aimant permanent » pourra être simplement désigné par « aimant ».

Lorsque ladite au moins une source de champ magnétique supplémentaire est un aimant, elle est avantageusement couplée à un élément ferromagnétique doux, autrement appelé « culasse » ou « circuit magnétique ». Cet élément ferromagnétique doux prolonge l'aimant et présente une perméabilité supérieure à 100 S.I (« système international », sans unité), et une saturation de 1,6 à 2,4 T. A l'inverse des éléments ferromagnétiques durs, les éléments ferromagnétiques doux présentent une aimantation rémanente et un champ coercitif faibles. Un tel élément ferromagnétique doux ne présente pas d'aimantation en l'absence de champ magnétique extérieur et permet de canaliser les lignes champ magnétique de l'aimant, et ainsi d'augmenter la valeur du champ magnétique généré par l'aimant.

Une bobine est constituée d'un enroulement de fil conducteur. Cet enroulement peut éventuellement être réalisé autour d'un matériau ferromagnétique que l'on appelle un noyau. Par opposition à l'aimant, une bobine n'émet un champ magnétique que lorsqu'une action particulière est appliquée, en l'occurrence lorsqu'un courant électrique est appliqué et traverse le fil conducteur. Aussi, dès lors que ce courant électrique n'est plus appliqué, plus aucun champ magnétique n'est généré.

Lorsque ladite au moins une source de champ magnétique supplémentaire est une bobine, elle est avantageusement une bobine planaire, c'est-à-dire que l'ensemble des spires sont dans au moins un plan, avantageusement dans 1 à 5 plans.

Selon un mode de réalisation de l'invention, ladite au moins une couche magnétique présente deux surfaces opposées entre elles que sont une surface de capture et une surface opposée, et ladite au moins une source de champ magnétique supplémentaire est une bobine planaire qui est accolée à la surface de capture de ladite au moins une couche magnétique. Ainsi, la bobine planaire recouvre au moins partiellement la surface de capture de ladite au moins une couche magnétique. Dans un tel cas, l'immobilisation des nanoparticules se fera au moins en partie sur la bobine planaire, au niveau des zones de capture.

Un électro-aimant produit un champ magnétique lorsqu'il est alimenté par un courant électrique : il convertit de l'énergie électrique en énergie magnétique. Il est constitué d'un bobinage et d'un noyau et/ou d'une ou plusieurs pièces polaires en matériau ferromagnétique doux. Aussi, comme dans le cas d'une bobine, un électro-aimant n'émet pas de champ magnétique lorsqu'aucun courant électrique ne le traverse.

Ladite au moins une source de champ magnétique supplémentaire peut être fixée ou non au support de capture.

Selon un mode de réalisation de l'invention, ladite au moins une source de champ magnétique supplémentaire est fixée au support de capture. Avantageusement, ladite fixation est réversible. Ainsi, cette fixation peut être réalisée par clippage au support de capture. Alternativement, ladite fixation est irréversible. Ainsi, cette fixation peut être réalisée par collage au support.

Selon un mode de réalisation de l'invention, ladite au moins une source de champ magnétique supplémentaire est configurée pour émettre un champ magnétique homogène.

Par homogène, il est entendu un champ magnétique dont le gradient est inférieur à 100 1.m$^{-1}$ suivant l'axe d'aimantation de ladite au moins une source de champ magnétique supplémentaire et inférieure à 150 1.m$^{-1}$ suivant un axe orthogonal à l'axe d'aimantation à la surface de ladite au moins une source de champ magnétique supplémentaire. Par « inférieur à 100 1.m$^{-1}$ » il est entendu dans l'invention 100 T.m$^{-1}$, 90 T.m$^{-1}$, 80 T.m$^{-1}$, 70 T.m$^{-1}$, 60 T.m$^{-1}$, 50 T.m$^{-1}$, 40 T.m$^{-1}$, 30 T.m$^{-1}$, 20 T.m$^{-1}$, 10 1.m$^{-1}$ ou 0 T.m$^{-1}$. Par « inférieur à 150 T.m$^{-1}$ » il est entendu dans l'invention 150 T.m$^{-1}$, 140 T.m$^{-1}$, 130 T.m$^{-1}$, 120 T.m$^{-1}$, 110 T.m$^{-1}$, 100 T.m$^{-1}$, 90 T.m$^{-1}$, 80 T.m$^{-1}$, 70 T.m$^{-1}$, 60 T.m$^{-1}$, 50 T.m$^{-1}$, 40 T.m$^{-1}$, 30 T.m$^{-1}$, 20 T.m$^{-1}$, 10 1.m$^{-1}$ ou 0 T.m$^{-1}$.

Le champ magnétique généré par ladite au moins une source supplémentaire peut prendre n'importe quelle direction, avantageusement sa direction est orthogonale à la surface du support, plus avantageusement sa direction et son sens sont les mêmes que ceux générés par ladite au moins une couche magnétique, à la surface du support de capture au niveau des jonctions entre premières et deuxièmes régions.

Ladite au moins une source de champ magnétique supplémentaire peut être disposée à n'importe quelle position autour du support de capture. Ainsi, ladite au moins une source supplémentaire peut aussi bien être disposée en regard de la surface de capture de ladite au moins une couche magnétique, ou bien en regard de sa seconde surface. Alternativement, ladite au moins une source supplémentaire peut être disposée à distance du support de capture, ni en regard de la première et de la deuxième surface de capture de ladite au moins une couche magnétique.

Ladite au moins une source de champ magnétique supplémentaire peut présenter une plus grande surface que la taille de la surface de ladite au moins une couche magnétique dont elle est en regard. En ce sens, lorsque ladite au moins une source de champ magnétique est disposée sous la couche magnétique, sa surface dépasse de part et d'autre de la couche magnétique. Par exemple, lorsque la source de champ magnétique supplémentaire est un assemblage d'aimants, une partie de la surface d'un ou d'au moins un des aimants n'est pas en regard de ladite au moins une couche magnétique ou au moins un aimant ne présente pas de surface en regard de ladite au moins une couche magnétique.

En effet, dans tous les cas, les effets importants apportés par cette au moins une source de champ magnétique sont d'une part l'aimantation des nanoparticules magnétiques, et d'autre part l'augmentation de l'amplitude des puits d'énergie, comme indiqué plus avant.

Ainsi, ladite au moins une source de champ magnétique supplémentaire est avantageusement configurée pour générer un champ magnétique de 1 mT à 400 mT au niveau des nanoparticules lors de l'utilisation du kit. Afin d'éviter un risque de désaimantation de la couche magnétique, il est avantageux que la valeur du champ coercitif de ladite au moins une source de champ magnétique supplémentaire soit au maximum de 90% de la valeur du champ coercitif de ladite au moins une couche magnétique.

Par « 1 mT et 400 mT » il est entendu dans l'invention 1 mT, 5mT, 10mT, 15mT, 20mT, 25mT, 30mT, 35mT, 40mT, 45mT, 50mT, 55mT, 60mT, 65mT, 70mT, 75mT, 80mT, 85mT, 90mT, 95mT, 100mT, 105mT, 110mT, 115mT, 120mT, 125mT, 130mT, 135mT, 140mT, 145mT, 150mT, 155mT, 160mT, 165mT, 170mT, 175mT, 180mT, 185mT, 190mT, 195mT, 200mT, 205mT, 210mT, 215mT, 220mT, 225mT, 230mT, 235mT, 240mT, 245mT, 250mT, 255mT, 260mT, 265mT, 270mT, 275mT, 280mT, 285mT, 290mT, 295mT, 300mT, 305mT, 310mT, 315mT, 320mT, 325mT, 330mT, 335mT, 340mT, 345mT, 350mT, 355mT, 360mT, 365mT, 370mT, 375mT, 380mT, 385mT, 390mT, 395mT, 400mT.

Avantageusement ladite au moins une source de champ magnétique est configurée pour générer un champ magnétique de 10mT à 400mT, plus avantageusement de 50mT à 200mT.

Selon un mode de réalisation de l'invention, ladite au moins une source de champ magnétique supplémentaire est configurée pour émettre un champ magnétique de manière continue.

Alternativement, ladite au moins une source de champ magnétique supplémentaire est configurée pour émettre un champ magnétique de manière pulsée. Avantageusement, la durée d'une impulsion est supérieure ou égale à 1 ms. Une telle durée permet d'augmenter l'aimantation des nanoparticules magnétiques pendant un laps de temps suffisamment long pour que leur déplacement en soit influencé, comparé à un simple mouvement Brownien.

L'invention concerne également un kit tel que défini précédemment où ladite au moins une couche magnétique présentant une surface de capture, ladite au moins une couche magnétique est au moins partiellement recouverte sur ladite surface de capture par une couche amagnétique.

Lorsqu'une telle couche amagnétique est présente, la capture et l'immobilisation des nanoparticules se font contre la surface de la couche amagnétique pour la partie de ladite au moins une couche magnétique revêtue, du fait que la surface dite « de capture » de ladite au moins une couche magnétique est revêtue.

La présence de cette couche amagnétique est avantageuse en ce qu'elle permet de retarder le déclenchement de l'attraction des nanoparticules par le support de capture au moment voulu, comme il sera vu plus loin en détail.

Selon un mode de réalisation de l'invention, le matériau de la couche amagnétique est choisi parmi la liste suivante : du verre, du silicium, un polymère de matériau plastique, du papier siliconé, un ruban adhésif, un alliage métallique non-magnétique tel que du dural ou du titane, du quartz, de la matière organique tel que du papier ou du bambou, du bois, de l'or ou une combinaison de ces matériaux.

Avantageusement, le polymère est choisi parmi la liste suivante : du polydiméthylsiloxane (PDMS), du polyméthacrylate de méthyle (PMMA), un polymère de cyclo-oléfines (COC/COP), du polycarbonate, du polyimide, du polychlorure de vinyle (PVC), du polyéthylène, du polypropylène, du silicone, du polyester, ou une combinaison de ces matériaux.

Selon un mode de réalisation, ladite couche amagnétique présente la même composition que celle de l'organe de soutien. Alternativement, ladite couche amagnétique présente une composition différente de celle de l'organe de soutien

Avantageusement, ladite au moins une source de champ magnétique ne présente pas de fluorescence. Encore plus avantageusement, elle est opaque afin de pas réfléchir la lumière.

Avantageusement, le matériau de la couche amagnétique est constitué par ou comprend un ruban adhésif présentant une superposition d'une couche de polychlorure de vinyle (PVC) et d'une couche adhésive ou d'une couche de polypropylène et d'une colle acrylique.

Selon un mode de réalisation de l'invention, ladite au moins une couche magnétique est recouverte sur au moins 1% de sa surface de capture par ladite couche amagnétique. Par « au moins 1% », il est entendu 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% ou 100%.

Avantageusement ladite au moins une couche magnétique est au recouverte sur au moins 30 % de sa surface de capture, plus avantageusement au moins 50% de sa surface de capture, encore plus avantageusement au moins 60% de sa surface de capture, avantageusement au moins 80% de sa surface de capture.

Selon un mode de réalisation de l'invention, la surface de capture de ladite au moins une couche magnétique est totalement recouverte par ladite couche amagnétique.

L'invention concerne également un kit tel que défini précédemment où ladite couche amagnétique présente une épaisseur de 1 à 300 μm. Par « 1 à 300 μm », il est entendu dans l'invention 1 μm, 2 μm, 3 μm, 4 μm, 5 μm, 6 μm, 7 μm, 8 μm, 9 μm, 10 μm, 11 μm, 12 μm, 13 μm, 14 μm, 15 μm, 16 μm, 17 μm, 18 μm, 19 μm, 20 μm, 21 μm, 22 μm, 23 μm, 24 μm, 25 μm, 26 μm, 27 μm, 28 μm, 29 μm, 30 μm, 31 μm, 32 μm, 33 μm, 34 μm, 35 μm, 36 μm, 37 μm, 38 μm, 39 μm, 40 μm, 41 μm, 42 μm, 43 μm, 44 μm, 45 μm, 46 μm, 47 μm, 48 μm, 49 μm, 50 μm, 51 μm, 52 μm, 53 μm, 54 μm, 55 μm, 56 μm, 57 μm, 58 μm, 59 μm, 60 μm, 61 μm, 62 μm, 63 μm, 64 μm, 65 μm, 66 μm, 67 μm, 68 μm, 69 μm, 70 μm, 71 μm, 72 μm, 73 μm, 74 μm, 75 μm, 76 μm, 77 μm, 78 μm, 79 μm, 80 μm, 81 μm, 82 μm, 83 μm, 84 μm, 85 μm, 86 μm, 87 μm, 88 μm, 89 μm, 90 μm, 91 μm, 92 μm, 93 μm, 94 μm, 95 μm, 96 μm, 97 μm, 98 μm, 99 μm, 100 μm, 101 μm, 102 μm, 103 μm, 104 μm, 105 μm, 106 μm, 107 μm, 108 μm, 109 μm, 110 μm, 111 μm, 112 μm, 113 μm, 114 μm, 115 μm, 116 μm, 117 μm, 118 μm, 119 μm, 120 μm, 121 μm, 122 μm, 123 μm, 124 μm, 125 μm, 126 μm, 127 μm, 128 μm, 129 μm, 130 μm, 131 μm, 132 μm, 133 μm, 134 μm, 135 μm, 136 μm, 137 μm, 138 μm, 139 μm, 140 μm, 141 μm, 142 μm, 143 μm, 144 μm, 145 μm, 146 μm, 147 μm, 148 μm, 149 μm, 150 μm, 151 μm, 152 μm, 153 μm, 154 μm, 155 μm, 156 μm, 157 μm, 158 μm, 159 μm, 160 μm, 161 μm, 162 μm, 163 μm, 164 μm, 165 μm, 166 μm, 167 μm, 168 μm, 169 μm, 170 μm, 171 μm, 172 μm, 173 μm, 174 μm, 175 μm, 176 μm, 177 μm, 178 μm, 179 μm, 180 μm, 181 μm, 182 μm, 183 μm, 184 μm, 185 μm, 186 μm, 187 μm, 188 μm, 189 μm, 190 μm, 191 μm, 192 μm, 193 μm, 194 μm, 195 μm, 196 μm, 197 μm, 198 μm, 199 μm, 200 μm, 201 μm, 202 μm, 203 μm, 204 μm, 205 μm, 206 μm, 207 μm, 208 μm, 209 μm, 210 μm, 211 μm, 212 μm, 213 μm, 214 μm, 215 μm, 216 μm, 217 μm, 218 μm, 219 μm, 220 μm, 221 μm, 222 μm, 223 μm, 224 μm, 225 μm, 226 μm, 227 μm, 228 μm, 229 μm, 230 μm, 231 μm, 232 μm, 233 μm, 234 μm, 235 μm, 236 μm, 237 μm, 238 μm, 239 μm, 240 μm, 241 μm, 242 μm, 243 μm, 244 μm, 245 μm, 246 μm, 247 μm, 248 μm, 249 μm, 250 μm, 251 μm, 252

µm, 253 µm, 254 µm, 255 µm, 256 µm, 257 µm, 258 µm, 259 µm, 260 µm, 261 µm, 262 µm, 263 µm, 264 µm, 265 µm, 266 µm, 267 µm, 268 µm, 269 µm, 270 µm, 271 µm, 272 µm, 273 µm, 274 µm, 275 µm, 276 µm, 277 µm, 278 µm, 279 µm, 280 µm, 281 µm, 282 µm, 283 µm, 284 µm, 285 µm, 286 µm, 287 µm, 288 µm, 289 µm, 290 µm, 291 µm, 292 µm, 293 µm, 294 µm, 295 µm, 296 µm, 297 µm, 298 µm, 299 µm ou 300 µm.

Avantageusement, ladite couche amagnétique présente une épaisseur de 1 à 150 µm, plus avantageusement 5 µm à 100 µm, encore plus avantageusement de 10 à 80 µm, avantageusement de 30 à 60 µm.

La couche amagnétique ne doit être ni trop fine ni trop épaisse. En effet, dans le cas où ladite couche amagnétique est trop fine, c'est-à-dire inférieure à 1 µm, elle n'a pas un impact significatif d'atténuation de l'attraction des nanoparticules par ladite au moins une couche magnétique.

Il est à noter que l'épaisseur de la couche va dépendre de la retentivité de ladite au moins une couche magnétique et de la largeur des premières et deuxièmes régions. L'homme du métier saura facilement adapter l'épaisseur de la couche magnétique en fonction de la retentivité et largeur des régions de ladite au moins une couche magnétique.

Typiquement, pour une couche magnétique de retentivité de 12000 µm.G et dont les premières et deuxièmes régions présentent une largeur de 50 µm, la couche amagnétique présentera avantageusement une épaisseur de 20 à 60 µm. Pour des couches magnétiques de plus faible retentivité, l'épaisseur de la couche magnétique devrait être réduite en fonction.

Dans le cas où la couche amagnétique serait trop épaisse (en fonction de l'épaisseur et des largeurs des régions de ladite au moins au moins une couche magnétique), les gradients de champ magnétique forts seront cachés par cette couche amagnétique. Ainsi le champ magnétique à la surface de ladite couche amagnétique sera homogène ou inexistant selon les cas. Les nanoparticules ne seront alors pas immobilisées, ou bien seront immobilisées de manière aléatoire, pas selon un motif particulier, à la surface de la couche amagnétique.

L'application du champ magnétique de ladite au moins une source de champ magnétique supplémentaire permet avantageusement dans un tel cas de « révéler » les gradients de champ magnétique forts à la surface de la couche amagnétique, de par l'augmentation de l'amplitude des puits d'énergie. Cet aspect de l'invention permet de « déclencher » la capture et l'immobilisation des nanoparticules au niveau des zones de capture, comme il sera vu en détail plus loin.

L'invention utilise également un support de capture comprenant plusieurs (de 1 à 100) canaux microfluidiques qui présentent d'une part soit une entrée commune à au **moins une** partie des canaux, soit une entrée indépendante pour chaque canal, et d'autre part en sortie un évent par canal ou bien un évent commun à au moins une partie des canaux. Ces canaux microfluidiques sont collés à une couche amagnétique, elle-même déposée sur une couche magnétique collée à un organe de soutien. La couche magnétique a été préalablement codée avec une aimantation selon le plan horizontal de ladite couche magnétique et une orientation variant de 180° d'une région à l'autre. Un aimant centimétrique ou millimétrique en Néodyme-Fer-Bore est utilisé comme source de champ magnétique supplémentaire afin appliquer un champ magnétique extérieur dont l'orientation et le sens sont les mêmes que ceux de la couche magnétique au niveau des jonctions entre premières et deuxièmes régions.

L'invention concerne également une méthode de capture d'une molécule contenue dans un échantillon, telle que définie dans la revendication 5, ladite méthode comprenant les étapes suivantes :

a) la mise en contact dudit échantillon avec des nanoparticules magnétiques telles que définies ci-dessus, de sorte à former au moins un complexe de capture entre ladite molécule et ledit au moins un élément de capture couplé auxdites nanoparticules magnétiques;

b) l'attraction dudit au moins un complexe de capture tel que formé lors de l'étape a) par le champ magnétique généré par au moins une couche magnétique d'un support de capture tel que défini précédemment, de sorte que ledit au moins un complexe de capture soit immobilisé contre ledit support de capture au niveau de la au moins une zone de capture telle que définie précédemment.

L'étape a) de la présente invention a pour but de complexer les molécules à capturer avec les nanoparticules via l'élément de capture, de sorte que ces molécules complexées puissent être indirectement attirées, via les nanoparticules, dans une étape subséquente b) au moyen de ladite au moins une couche magnétique et, *in fine,* être capturées par immobilisation contre le support.

Pour la suite de la description, un complexe de capture pourra être simplement désigné par le terme « complexe ».

Selon un mode de réalisation de l'invention, ledit élément de capture est un anticorps ou un antigène de sorte que ledit au moins un complexe de capture formé lors de l'étape a) est un complexe immun.

Par « échantillon », il est entendu dans l'invention tout fluide simple ou complexe.

**Par** « **fluide** complexe », on entend dans l'invention un mélange qui présente une coexistence entre deux phases : solide-liquide (suspensions ou solutions de macromolécules telles que des polymères), solide-gaz (granulaire), liquide-gaz (mousses) ou liquide-liquide (émulsions). Les fluides complexes s'écartent de la relation newtonienne linéaire classique entre la contrainte et le taux de cisaillement. Ils présentent des réponses mécaniques inhabituelles à la contrainte ou à la déformation appliquée en raison des contraintes géométriques que la coexistence des phases impose. La réponse mécanique comprend des transitions entre un comportement de type solide et un comportement de type fluide ainsi que des fluctuations. Notamment, l'échantillon peut être un fluide biologique tel que du sang, de l'urine, de la lymphe, du plasma, du sérum, de la salive, des larmes, du sperme, des sécrétions vaginales, du pus d'une plaie, du fluide gastrique ou encore du liquide cérébro-spinal. L'échantillon peut également être un milieu utilisé dans des bioprocédés, tel qu'un milieu de culture, un milieu de culture purifié ou clarifié.

Par « simple fluide », on entend dans l'invention un fluide newtonien dont le comportement mécanique est caractérisé par une seule fonction de la température, la viscosité, une mesure du "glissement" du fluide. Une contrainte appliquée sur un fluide simple est directement proportionnelle à la vitesse de déformation. Notamment, l'échantillon peut être de l'eau déionisée. L'eau déionisée présente l'avantage de limiter fortement, voire d'empêcher, la formation d'amas de nanoparticules.

Selon un mode de réalisation de l'invention, l'échantillon une fois mis en contact avec les nanoparticules lors de l'étape a) est disposé au niveau du support de capture afin de permettre l'attraction dudit au moins un complexe de capture lors de l'étape b).

Ainsi, l'étape de mise en contact et celle d'attraction sont réalisées séparément, de sorte que les nanoparticules ne sont pas attirées par ladite au moins une couche magnétique lors de leur mise en contact avec l'échantillon. Ceci permet avantageusement d'obtenir une répartition homogène des nanoparticules dans l'échantillon et donc une complexation plus efficace des éléments de capture avec la molécule à capturer.

Alternativement, l'échantillon est tout d'abord disposé au niveau support avant sa mise en contact avec les nanoparticules lors de l'étape a). Ainsi, l'exécution des étapes a) et b) est réalisée au même endroit, c'est-à-dire au niveau du support. Ce mode de réalisation est d'un grand intérêt dans certaines applications, notamment parce qu'il ne nécessite pas de manipulation de fluide via des actionneurs de types micro-pompes ou micro-vannes.

Selon un mode de réalisation de l'invention, entre l'étape a) et l'étape b) est opérée une sonication du mélange comprenant les complexes de nanoparticules-molécules en suspension à capturer, dans le but de détruire tout agrégat de complexes qui pourrait avoir été formés. Ces agrégats perturberaient en effet l'étape de quantification vue plus loin. Cette sonication peut notamment être réalisée à une fréquence d'au moins 10000Hz. La sonication peut être continue ou pulsée. Lorsque la sonication est pulsée, la durée de chaque sonication peut aller de 200 à 700 millisecondes, notamment de 300 à 600 millisecondes, particulièrement de 500 millisecondes, espacée de 1 à 6 secondes, notamment de 1 à 4 secondes, particulièrement de 2 secondes. Ces conditions de sonication permettent d'assurer la fonction de destruction des agrégats, tout en évitant une surchauffe du milieu qui pourrait dénaturer les éléments de captures et les molécules à capturer et les éléments de détection.

Lors de l'étape a), la concentration des nanoparticules est avantageusement de $10^6$ à $10^{11}$ particules/ml. Par « de $10^6$ à $10^{11}$ particules/ml », il est entendu dans l'invention, $10^6$ particules/ml, $10^7$ particules/ml, $10^8$ particules/ml, $10^9$ particules/ml, $10^{10}$ particules/ml et $10^{11}$ particules/ml.

Une concentration minimale de $10^6$ particules/ml apporte une concentration suffisante pour une capture efficace des molécules à capturer dispersées dans l'échantillon. En outre, une concentration maximale de $10^{11}$ particules/ml permet d'éviter une agglomération des nanoparticules trop importante (amas de diamètre inférieur ou égal 15 μm), qui d'une part perturberait la liaison entre l'élément de capture et la molécule à capturer, et d'autre part aurait un impact négatif sur la quantification de la molécule capturée. Une forte concentration en nanoparticules serait également responsable d'un écrantage des champs magnétiques, c'est-à-dire leur atténuation, et aurait un impact négatif sur l'attraction des nanoparticules. Par « amas de diamètre inférieur ou égal à 15 μm », il est entendu dans l'invention 15 μm, 10 μm, 5 μm, 4, μm, 3 μm, 2 μm, 1 μm, 0,5 μm et 0,2 μm.

Selon un mode de réalisation, l'étape a) comprend en outre la mise en contact de l'échantillon avec un élément de détection de ladite molécule.

Cet élément de détection présente un marqueur qui peut être fluorescent, luminescent ou coloré de sorte à être reconnu par un moyen de détection approprié. Ce marqueur peut également être une enzyme ayant des propriétés d'oxydo-réduction.

Lors de l'étape a) il est ainsi formé un complexe dit « sandwich » formé d'une nanoparticule magnétique, de l'élément de capture, de la molécule à capturer, et de l'élément de détection. La molécule à capture se retrouve alors entourée, prise en « sandwich », par l'élément de capture et l'élément de détection.

La formation de ces complexes « sandwich », où l'élément de détection est fixé à la molécule à capturer dès la première étape, est rendue possible grâce aux bonnes caractéristiques de diffusion dans le mélange des nanoparticules magnétiques. Ainsi, la formation de tels complexes serait rendue plus difficile avec des microparticules magnétiques.

L'étape de sonication mentionnée plus haut peut également être opérée dans ce mode de réalisation dans les mêmes conditions afin d'éviter tout agrégat de complexes « sandwich ».

Alternativement, l'élément de détection peut être mis en présence de la molécule à capturer lors d'une étape c), faisant suite à l'immobilisation contre le support des complexes formés d'une nanoparticule, d'un élément de capture et d'une molécule à capture. Aussi, les complexes dits « sandwich » tels que décrit ci-dessus sont-ils formés *a posteriori* lors cette étape c).

*In fine,* il est obtenu à la fin de l'étape a) un mélange comprenant des complexes de nanoparticules, éléments de capture et molécules à capturer, ou des complexes de nanoparticules, éléments de captures, molécules à capturer et élément de détection si un tel élément de détection est présent. Dans ce mélange il peut rester des molécules à capturer seules et des nanoparticules couplées aux éléments de capture seules, et optionnellement des éléments de détection seuls.

Ledit mélange peut être déposé, par exemple au moyen d'une pipette, sur un support de capture afin d'immobiliser les nanoparticules lors de l'étape b). Ledit mélange peut également être injecté dans le support de capture, dans le cas où ce support de capture est une chambre par exemple.

Une fois ledit mélange disposé au niveau du support, l'ensemble des nanoparticules magnétiques (complexées ou non) va être attiré par ladite au moins une couche magnétique et venir s'immobiliser contre le support, et plus précisément contre la surface de capture de ladite au moins une couche magnétique.

Lors de leur immobilisation, les nanoparticules magnétiques (complexées ou non) ne sont pas réparties de manière aléatoire contre le support, et plus précisément contre la surface de capture de ladite au moins une couche magnétique.

En effet, les nanoparticules sont immobilisées au niveau des zones de capture telles que définies dans l'invention. Aussi, seule une fraction minoritaire des nanoparticules, inférieure à 15%, seront soit immobilisées en dehors de ces zones de capture, soit ne seront pas capturées.

Ainsi, les nanoparticules sont réparties contre le support, et plus précisément contre la surface de capture de ladite au moins une couche magnétique, selon un motif particulier défini par l'ensemble des jonctions des premières et deuxièmes régions.

Cette répartition est très intéressante car elle permet de déterminer où seront capturées les nanoparticules, ce qui permet une quantification directe desdites molécules capturées sans passer par une étape de lavage.

L'organisation des premières et deuxièmes régions de ladite au moins une couche magnétique permet de faire une détection et une quantification sans lavage du support directement après l'immobilisation des complexes de capture et la liaison avec les éléments de détection ou l'immobilisation des complexes de capture « sandwich ».

Pour cela il convient tout d'abord de déterminer la quantité de marquage au niveau des zones de capture, puis celle en dehors des zones de capture.

L'homme du métier saura aisément adapter les moyens de détermination de la quantité de marquage à mettre en œuvre en fonction du type de marqueur couplé aux éléments de détection. Notamment, ces moyens peuvent être choisis parmi un spectrophotomètre en mode scanning, un microscope par épifluorescence, un microscope confocal, un micrococoscope biphotonique, la mesure l'activité d'oxydoréduction d'une enzyme, etc.

Par exemple, dans le cadre d'un marquage par fluorescence, l'homme du métier aura avantage à utiliser un microscope à fluorescence équipé d'un cube "GFP" (excitation 460-490 nm) ou d'un cube « ACP » (excitation 650 nm - émission 660 nm) couplé à une caméra à transfert de charge (CCD) ou CMOS, comme il est présenté dans la partie « Exemples » de la présente description.

La quantité de marquage en dehors des zones de capture correspond à du « bruit de fond » (background signal). Autrement dit, ce marquage correspond aux éléments de détection ne s'étant pas couplé aux complexes de capture et à la fraction minoritaire des complexes immobilisés en dehors des zones de capture ainsi qu'à l'effet de la matrice (signal résiduel du milieu)

De manière à obtenir la quantité de marquage spécifique émise au niveau des zones de capture, on soustrait la quantité de marquage obtenue en dehors de celle des zones de capture à celle obtenue au niveau des zones de capture.

L'invention concerne également une méthode de capture telle que définie précédemment où l'attraction dudit au moins un complexe de capture lors de l'étape b) est réalisée par l'action conjointe du champ magnétique généré par ladite au moins une couche magnétique et par un champ magnétique généré par au moins une source de champ magnétique supplémentaire telle que définie précédemment.

Comme il a été mentionné ci-dessus, ladite au moins une source de champ magnétique supplémentaire permet d'augmenter, voire de saturer, l'aimantation des nanoparticules magnétiques et d'autre part d'augmenter l'amplitude des puits d'énergie, et ainsi d'accélérer l'attraction des nanoparticules par ladite au moins une couche magnétique.

Par ailleurs comme discuté plus haut, le champ magnétique supplémentaire permet de renforcer certaines zones de capture et ainsi favorise une capture localisée, qui favorise dès lors la détection sans lavage de la molécule capturée, comme décrit plus haut.

Cette action conjointe permet également d'éviter l'utilisation d'une évaporation du solvant de l'échantillon visant à rapprocher les nanoparticules du support de capture et ainsi accélérer leur capture. Une telle évaporation nécessite en effet le chauffage de l'échantillon ou un temps très conséquent d'attente, ce qui pourrait avoir des répercussions négatives sur la liaison entre l'élément de capture / l'élément de détection et la molécule à capturer et donc sur la quantification de la molécule à capturer.

L'attraction des nanoparticules est donc réalisée par l'action conjointe de ladite au moins une source de champ magnétique et de ladite au moins une couche magnétique, chacune exerçant une action et une fonction différente.

De par sa fonction et son action, ladite au moins une source de champ magnétique peut être disposée n'importe où par rapport au support de capture, comme il est mentionnée ci-avant.

Ainsi, ladite au moins une source supplémentaire peut aussi bien être disposée

- en regard de la surface de capture de ladite au moins une couche magnétique et/ou de la couche amagnétique,
- en regard de la surface opposée ou de l'organe de soutien, ou bien
- à n'importe quelle autre position, décalée latéralement par exemple.

Une aimantation significative des nanoparticules est avantageusement atteinte par l'application d'un champ magnétique à leur niveau de 1mT à 400mT, avantageusement 10mT à 400mT, plus avantageusement de 50mT à 200mT.

L'action de ladite au moins une source de champ magnétique supplémentaire peut être réalisée tout au long des étapes a) et b).

Avantageusement elle est déclenchée lors de l'étape b). Aussi, lors de l'étape a) et avant le déclenchement de l'action de ladite au moins une source de champ magnétique supplémentaire, son champ magnétique au niveau des nanoparticules magnétiques est insuffisant voire nul.

Le déclenchement de l'action de ladite au moins une source de champ supplémentaire lors de l'étape b) est assuré par la génération d'un champ magnétique au niveau des nanoparticules magnétiques de 1mT à 400mT, avantageusement 10mT à 400mT, plus avantageusement de 50mT à 200mT.

De manière à déclencher l'action de ladite au moins une source de champ magnétique supplémentaire, cette dernière peut selon une première alternative être approchée du support de capture. Pour ce mode de réalisation, ladite au moins une source de champ magnétique supplémentaire est avantageusement un aimant permanent.

Selon une seconde alternative, ce déclenchement est réalisé par le passage d'un courant électrique dans ladite source de champ magnétique supplémentaire. Pour ce mode de réalisation, ladite au moins une source de champ magnétique supplémentaire est avantageusement une bobine ou un électro-aimant, et le déclenchement est réalisé par le passage d'un courant électrique dans ladite source de champ magnétique supplémentaire.

Avantageusement, même si l'action de ladite au moins une source de champ magnétique supplémentaire est arrêtée en fin d'étape b), par exemple soit en éloignant cette source, soit en arrêtant le passage du courant électrique la traversant, les nanoparticules capturées restent en place contre le support de capture au niveau des zones de capture. Ainsi, il est possible de déplacer le support de capture dans un lieu approprié pour réaliser la détection du marqueur des éléments de détection, sans que la position de ces nanoparticules n'en soit impactée et donc qu'une détection directe sans étape de lavage ne soit compromise.

L'invention concerne également une méthode de capture telle que définie précédemment dans laquelle le support de capture comprend en outre une couche amagnétique telle que définie précédemment, et où l'attraction dudit au moins un complexe de capture par le support de capture lors de l'étape b) est déclenchée au moyen du champ magnétique de ladite au moins une source de champ magnétique supplémentaire.

Dans ce mode de réalisation, la présence d'une couche amagnétique diminue la possibilité, voire empêche, que les nanoparticules ne soient attirées par le seul champ magnétique généré par ladite au moins une couche magnétique.

Ainsi, avantageusement,

- d'une part le champ magnétique généré par l'ensemble de ladite au moins une couche magnétique et de ladite au moins une source supplémentaire présente au moins une variation de son intensité d'au moins 0,1 mT à une distance d'au moins 1 μm de la surface de capture de la dite couche amagnétique, ladite au moins une variation de son intensité définissant un maximum et un minimum de la norme de l'intensité dudit champ magnétique, de sorte à définir au niveau dudit maximum de la norme dudit champ magnétique une zone de capture des nanoparticules magnétiques sur le support de capture, et
- d'autre part le champ magnétique généré par ladite au moins une couche magnétique ne présente pas au moins une variation de son intensité d'au moins 0,1 mT à une distance d'au moins 1 μm de la surface de ladite couche amagnétique, de sorte que le champ magnétique généré par seulement ladite au moins une couche magnétique ne permette pas de définir une zone de capture des nanoparticules magnétiques sur la surface de ladite couche amagnétique.

La surface du support correspondant à sa surface contre laquelle les nanoparticules sont immobilisées.

Aussi, l'attraction des nanoparticules magnétiques lors de l'étape b) est-elle subordonnée à l'action de ladite au moins une source de champ magnétique sur ces nanoparticules, c'est à dire par

- une augmentation significative de l'aimantation de ces particules au moyen du champ magnétique généré par cette source, et/ou
- par une augmentation de l'amplitude des puits d'énergie.

Cet aspect de l'invention est très intéressant, car il permet d'optimiser l'exécution des étapes a) et b) en un même endroit, c'est-à-dire au niveau du support de capture, en diminuant, voire en annulant, la possibilité que les nanoparticules puissent être attirées par l'action seule de ladite au moins une couche magnétique lors de l'étape a).

Du fait de la présence d'une couche amagnétique, au moins une partie des nanoparticules (complexées ou non) ne vont pas être immobilisées contre ladite au moins une couche magnétique, mais contre cette couche amagnétique. Plus précisément, la couche amagnétique présentant une surface de capture et une surface opposée en regard de ladite au moins une couche magnétique, au moins une partie des nanoparticules sont immobilisées lors de l'étape b) contre la première surface de capture de la couche amagnétique.

L'invention concerne également une méthode de capture telle que définie précédemment dans laquelle l'échantillon est disposé au niveau du support de capture avant l'étape a) de mise en contact avec les nanoparticules magnétiques.

Avantageusement, l'action de ladite au moins une source de champ magnétique est déclenchée lors de de l'étape b).

Aussi, lors de l'étape a), l'aimantation des nanoparticules magnétiques et/ou l'amplitude des puits d'énergie de ladite au moins une couche magnétique ne sont pas suffisants pour permettre l'attraction desdites nanoparticules par l'action de seulement ladite au moins une couche magnétique recouverte par ladite couche amagnétique. Ainsi, le mélange des nanoparticules avec l'échantillon est peu, voire pas, perturbé par une attraction précoce par ladite au moins une source magnétique supplémentaire.

De manière surprenante, les inventeurs ont découvert qu'en dépit de la présence d'une couche amagnétique et de son impact sur l'attractivité de ladite au moins une couche magnétique sur les nanoparticules, si l'action du champ magnétique généré par ladite au moins une source de champ magnétique supplémentaire est arrêtée en fin d'étape b), par exemple soit en éloignant cette source, soit en arrêtant le courant électrique, les nanoparticules immobilisées restent en place contre le support au niveau des zones de capture.

Une explication du phénomène serait que les gradients de champs magnétique produits par ladite au moins une couche magnétique et présents à la surface de la couche amagnétique seraient suffisants pour maintenir en place les nanoparticules contre ladite couche amagnétique. Une explication complémentaire serait qu'il y aurait une adsorption, c'est-à-dire un collage par voie chimique/physique, entre d'une part les nanoparticules magnétiques qui se sont agglomérées entre elles et d'autre part les particules absorbées avec la surface de capture de la couche amagnétique.

Ainsi, ici encore, il est possible de déplacer le support de capture dans un lieu approprié pour réaliser la détection du marqueur des éléments de détection, sans que la position de ces particules n'en soit impactée et donc qu'une détection directe sans étape de lavage ne soit compromise.

L'invention concerne également une méthode de capture telle que définie précédemment comprenant une étape subséquente c) de déplacement des nanoparticules magnétiques, qui ont été capturées contre le support, dans une zone de récupération.

Ce déplacement est réalisé grâce à une technique dite « bielle-manivelle » dans laquelle :

- la manivelle est le champ magnétique généré par les régions magnétiques de ladite au moins une couche magnétique ; on note ce champ magnétique b(R) (R étant un point de l'espace). Ladite au moins une couche magnétique présentant une variation de l'intensité du champ magnétique généré, de sorte à créer des minima d'énergie magnétique,
- la bielle est un champ magnétique spatialement homogène (en direction et en intensité) généré par ladite au moins une source de champ magnétique supplémentaire. Dans cette application, ledit champ magnétique peut être modifié dans son amplitude et son orientation.

Le champ magnétique « homogène » généré par la bielle est appliqué sur toute la zone où le champ magnétique de la manivelle est généré. Ainsi le champ magnétique généré par la bielle coexiste dans l'espace avec le champ magnétique généré par ladite au moins une couche magnétique, de sorte que leurs valeurs vectorielles se superposent linéairement dans un milieu de perméabilité relative égale à un (tel que l'air ou l'eau).

Du fait que, le champ magnétique généré par la bielle est modulable, il est possible lors de l'étape c) de le faire tourner d'au moins 1° dans le sens horaire ou antihoraire autour d'au moins un axe de rotation et/ou d'amplifier ledit champ magnétique. Par conséquent, il est possible de modifier temporellement le champ magnétique de la bielle. Ainsi on note le champ magnétique de la bielle B(t) (t étant le temps).

Dès lors, le champ magnétique de la manivelle varie dans l'espace, mais pas dans le temps et celui de la bielle varie dans le temps.,.

Par conséquent, dans la zone où la bielle et la manivelle sont appliquées, le champ magnétique total $B_T$ vaut la somme vectorielle de b et B, soit $B_T(R,t) = b(R) + B(t)$.

De cette manière il est possible lors de l'étape c) de modifier la position des zones de capture des nanoparticules magnétiques contre le support.

Par « de 1° à 360° », il est entendu dans l'invention 1°, 2°, 3°, 4°, 5°, 6°, 7°, 8°, 9°, 10°, 11°, 12°, 13°, 14°, 15°, 16°, 17°, 18°, 19°, 20°, 21°, 22°, 23°, 24°, 25°, 26°, 27°, 28°, 29°, 30°, 31°, 32°, 33°, 34°, 35°, 36°, 37°, 38°, 39°, 40°, 41°, 42°, 43°, 44°, 45°, 46°, 47°, 48°, 49°, 50°, 51°, 52°, 53°, 54°, 55°, 56°, 57°, 58°, 59°, 60°, 61°, 62°, 63°, 64°, 65°, 66°, 67°, 68°, 69°, 70°, 71°, 72°, 73°, 74°, 75°, 76°, 77°, 78°, 79°, 80°, 81°, 82°, 83°, 84°, 85°, 86°,

87°, 88°, 89°, 90°, 91°, 92°, 93°, 94°, 95°, 96°, 97°, 98°, 99°, 100°, 101°, 102°, 103°, 104°, 105°, 106°, 107°, 108°, 109°, 110°, 111°, 112°, 113°, 114°, 115°, 116°, 117°, 118°, 119°, 120°, 121°, 122°, 123°, 124°, 125°, 126°, 127°, 128°, 129°, 130°, 131°, 132°, 133°, 134°, 135°, 136°, 137°, 138°, 139°, 140°, 141°, 142°, 143°, 144°, 145°, 146°, 147°, 148°, 149°, 150°, 151°, 152°, 153°, 154°, 155°, 156°, 157°, 158°, 159°, 160°, 161°, 162°, 163°, 164°, 165°, 166°, 167°, 168°, 169°, 170°, 171°, 172°, 173°, 174°, 175°, 176°, 177°, 178°, 179°, 180°, 181°, 182°, 183°, 184°, 185°, 186°, 187°, 188°, 189°, 190°, 191°, 192°, 193°, 194°, 195°, 196°, 197°, 198°, 199°, 200°, 201°, 202°, 203°, 204°, 205°, 206°, 207°, 208°, 209°, 210°, 211°, 212°, 213°, 214°, 215°, 216°, 217°, 218°, 219°, 220°, 221°, 222°, 223°, 224°, 225°, 226°, 227°, 228°, 229°, 230°, 231°, 232°, 233°, 234°, 235°, 236°, 237°, 238°, 239°, 240°, 241°, 242°, 243°, 244°, 245°, 246°, 247°, 248°, 249°, 250°, 251°, 252°, 253°, 254°, 255°, 256°, 257°, 258°, 259°, 260°, 261°, 262°, 263°, 264°, 265°, 266°, 267°, 268°, 269°, 270°, 271°, 272°, 273°, 274°, 275°, 276°, 277°, 278°, 279°, 280°, 281°, 282°, 283°, 284°, 285°, 286°, 287°, 288°, 289°, 290°, 291°, 292°, 293°, 294°, 295°, 296°, 297°, 298°, 299°, 300°, 301°, 302°, 303°, 304°, 305°, 306°, 307°, 308°, 309°, 310°, 311°, 312°, 313°, 314°, 315°, 316°, 317°, 318°, 319°, 320°, 321°, 322°, 323°, 324°, 325°, 326°, 327°, 328°, 329°, 330°, 331°, 332°, 333°, 334°, 335°, 336°, 337°, 338°, 339°, 340°, 341°, 342°, 343°, 344°, 345°, 346°, 347°, 348°, 349°, 350°, 351°, 352°, 353°, 354°, 355°, 356°, 357°, 358°, 359° ou 360°.

Il est alors obtenu un résultat de sommation vectorielle des champs magnétiques de la bielle et de la manivelle différent de celui obtenu en fin d'étape b), et la localisation des maximums de la norme de l'intensité du champ magnétique généré par l'ensemble bielle et manivelle est déplacé, de sorte que les zones de capture sont décalées par rapport à cette étape b). Ainsi, les nanoparticules, attirées par les gradients de champ magnétique générés, sont déplacées en même temps jusqu'à être positionnées à la nouvelle position des zones de capture.

Avantageusement, l'étape c) est répétée dans le même sens de rotation jusqu'à ce que les nanoparticules, étant progressivement déplacées dans la même direction, atteignent la zone de récupération.

En répétant l'étape c), il est obtenu un effet « tapis roulant », où les nanoparticules sont déplacées dans la même direction le long de la surface du support de capture vers une zone de récupération. La molécule à capturer étant complexée avec les nanoparticules, elle est également récupérée dans cette zone de récupération.

*In fine,* grâce à l'invention, il est possible de récupérer la molécule à capturer sans appliquer un flux fluidique sur le support qui déplacerait l'ensemble des molécules contenues dans le mélange.

L'invention concerne en outre une utilisation d'un kit tel que défini précédemment pour la capture d'une molécule contenue dans un échantillon, telle que définie dans la revendication 9, avantageusement pour la capture et la détection d'une molécule contenue dans un échantillon.

Il est décrit également une utilisation d'une couche magnétique pour attirer des nanoparticules présentant comme plus grande dimension une dimension inférieure à 1 μm, ladite couche magnétique comprenant une juxtaposition, possiblement répétée, d'au moins une première et une deuxième région, la première région comprenant des grains magnétiques polarisés dans une première direction, et la deuxième région comprenant des grains magnétiques non polarisés ou polarisés dans une deuxième direction différente de la première direction de polarisation des particules magnétiques de la première région, de sorte à ce que ladite une couche magnétique génère un champ magnétique présentant au moins une variation de son intensité d'au moins 0,1 mT à une distance d'au moins 1 μm de ladite au moins une couche magnétique, ladite au moins une variation de son intensité définissant un maximum et un minimum de la norme de l'intensité dudit champ magnétique, de sorte à définir au niveau dudit maximum de la norme dudit champ magnétique une zone de capture des nanoparticules magnétiques sur ladite couche magnétique, et lesdites nanoparticules étant chacune couplée à au moins un élément de capture d'une molécule.

Il est aussi décrit un autre kit de capture d'une molécule contenue dans un échantillon comprenant :

a) des nanoparticules magnétiques présentant comme plus grande dimension une dimension inférieure à 1 μm, lesdites nanoparticules étant chacune couplée à au moins un élément de capture, ledit au moins un élément de capture se liant spécifiquement à ladite molécule, et

b) un support de capture desdites nanoparticules magnétiques comprenant ou étant constitué essentiellement d'au moins une couche magnétique, ladite couche magnétique comprenant une juxtaposition, possiblement répétée, d'au moins une première et une deuxième région, la première région comprenant des particules magnétiques polarisées dans une première direction, et la deuxième région comprenant des particules magnétiques non polarisées ou polarisées dans une deuxième direction différente à la première direction de polarisation des particules magnétiques de la première région, de sorte à ce que ladite au moins une couche magnétique génère un champ magnétique ne présentant pas au moins une variation de son intensité d'au moins 0,1 mT à une distance d'au moins 1 μm de ladite au moins une couche magnétique,

c) au moins une source de champ magnétique supplémentaire, de sorte que le champ magnétique généré par l'ensemble de ladite au moins une couche magnétique et de ladite au moins une source supplémentaire présente au moins une variation de son intensité d'au moins 0,1 mT à une distance d'au moins 1 μm de ladite au moins une couche magnétique,

ladite au moins une variation de son intensité définissant un maximum et un minimum de la norme de l'intensité dudit champ magnétique, de sorte à définir au niveau dudit maximum de la norme dudit champ magnétique une zone de capture des nanoparticules magnétiques sur le support de capture.

Avantageusement, ladite source supplémentaire est externe à ladite au moins une couche magnétique.

Avantageusement encore, ladite au moins une couche magnétique présentant une surface de capture, ladite au moins une couche magnétique est au moins partiellement recouverte sur ladite surface de capture par une couche amagnétique, et le champ magnétique généré par l'ensemble de ladite au moins une couche magnétique et ladite au moins une source supplémentaire présente au moins une variation de son intensité d'au moins 0,1 mT à une distance d'au moins 1 μm de la surface de capture de la dite couche amagnétique, ladite au moins une variation de son intensité définissant un maximum et un minimum de la norme de l'intensité dudit champ magnétique, de sorte à définir au niveau dudit maximum de la norme dudit champ magnétique une zone de capture des nanoparticules magnétiques sur le support de capture.

L'ensemble des caractéristiques décrites ci-dessus relatives au support de capture, à la source de champ magnétique supplémentaire, et aux nanoparticules concernant le kit décrit plus haut s'applique *mutatis mutandis* au présent kit.

L'invention concerne par ailleurs une méthode de capture d'une molécule contenue dans un échantillon, ladite méthode comprenant les étapes suivantes :

a) la mise en contact dudit échantillon avec des nanoparticules magnétiques telles que définies ci-dessus, de sorte à former au moins un complexe de capture entre ladite molécule et ledit au moins un élément de capture couplé auxdites nanoparticules magnétiques;

b) l'attraction dudit au moins un complexe de capture tel que formé lors de l'étape a) par le champ magnétique généré par un ensemble d'au moins une couche magnétique d'un support de capture et d'au moins une source de champ magnétique supplémentaire tels que définis précédemment, de sorte que ledit au moins un complexe de capture soit immobilisé contre ledit support de capture au niveau de la au moins une zone de capture telle que définie précédemment.

L'invention concerne également une utilisation d'un kit tel que défini précédemment pour la capture d'une molécule contenue dans un échantillon, avantageusement pour la capture et la détection d'une molécule contenue dans un échantillon.

Il est enfin décrit une utilisation d'un ensemble comprenant une couche magnétique et une source de champ supplémentaire pour attirer des nanoparticules présentant comme plus grande dimension une dimension inférieure à 1 μm, ladite couche magnétique comprenant une juxtaposition, possiblement répétée, d'au moins une première et une deuxième région, la première région comprenant des grains magnétiques polarisés dans une première direction, et la deuxième région comprenant des grains magnétiques non polarisés ou polarisés dans une deuxième direction différente de la première direction de polarisation des particules magnétiques de la première région, de sorte que le champ magnétique généré par l'ensemble de ladite une couche magnétique et de ladite source supplémentaire présente au moins une variation de son intensité d'au moins 0,1 mT à une distance d'au moins 1 μm de ladite au moins une couche magnétique, ladite au moins une variation de son intensité définissant un maximum et un minimum de la norme de l'intensité dudit champ magnétique, de sorte à définir au niveau dudit maximum de la norme dudit champ magnétique une zone de capture des nanoparticules magnétiques sur ladite couche magnétique.

Avantageusement, lesdites nanoparticules sont chacune couplée à au moins un élément de capture d'une molécule.

## Brève description des figures

La figure 1 représente une capture de nanoparticules par une couche magnétique selon l'invention. Le système de capture est représenté en vue en coupe. L'intensité des champs magnétiques émis par la couche magnétique est définie par un code couleur dont l'échelle est présentée à droite de la vue en coupe (en T). Les flèches dans la couche magnétique représentent la direction de la polarisation des grains la composant.

La figure 2 représente une photographie d'une capture par le système représenté en figure 1. Sur cette photographie, les points blancs représentent les nanoparticules.

La figure 3 représente une capture de nanoparticules par une couche magnétique selon l'invention, ladite couche étant recouverte d'une couche amagnétique. Le système de capture est représenté en vue en coupe. L'intensité des champs magnétiques générés par la couche magnétique est définie par un code couleur dont l'échelle est présentée à droite de la vue en coupe (en T). Les flèches partant des nanoparticules représentent la direction du mouvement des nanoparticules attirées par la couche magnétique. Les flèches dans la couche magnétique représentent la direction de la polarisation des grains la composant.

La figure 4 représente une photographie d'une capture par le système représenté en figure 3. Sur cette photographie, les points blancs représentent les nanoparticules.

La figure 5a représente une capture de nanoparticules par une couche magnétique selon l'invention, ladite couche étant recouverte d'une couche amagnétique et la capture étant réalisée en présence du champ magnétique d'une source supplémentaire. Le système de capture est représenté en vue en coupe. La direction du champ magnétique généré par la source supplémentaire est représentée par la flèche blanche à bord noire au-dessus de la vue en coupe et est perpendiculaire à la couche magnétique. L'intensité des champs magnétiques générés par la couche magnétique est définie par un code couleur dont l'échelle est présentée à droite de la vue en coupe (en T). Les flèches partant des nanoparticules représentent la direction du mouvement des nanoparticules attirées par la couche magnétique. Les flèches dans la couche magnétique représentent la direction de la polarisation des grains la composant.

La figure 5b représente une capture de nanoparticules par une couche magnétique selon l'invention, ladite couche étant recouverte d'une couche amagnétique et la capture étant réalisée en présence du champ magnétique d'une source supplémentaire. Le système de capture est représenté en vue en coupe. La direction du champ magnétique généré par la source supplémentaire est représentée par la flèche blanche à bord noire au-dessus de la vue en coupe et est parallèle à la couche magnétique. L'intensité des champs magnétiques totaux (généré par la couche magnétique et la source supplémentaire) est définie par un code couleur dont l'échelle est présentée à droite de la vue en coupe (en T). Les flèches partant des nanoparticules représentent la direction du mouvement des nanoparticules attirées par la couche magnétique. Les flèches dans la couche magnétique représentent la direction de la polarisation des grains la composant.

La figure 6 représente une photographie d'une capture par le système représenté en figure 5a. Sur cette photographie, les points blancs représentent les nanoparticules.

La figure 7 représente le pourcentage de nanoparticules capturées (axes X), en fonction du temps (axes Y) pour chaque capture des captures présentées en figures 1 à 3. Le temps exprimé en minutes. La courbe A correspondent à la cinétique de capture représentée en figure 5a, la courbe B correspondent à la cinétique de capture représentée en figure 1, et la courbe C correspondent à la cinétique capture représentée en figure 3.

La figure 8 représente des anticorps de capture de détection anti-souris portant un fluorochrome et couplés à des complexes de nanoparticules greffées par des anticorps de capture anti-ovalbumine de souris. Il est également une nanoparticule greffée avec de l'ovalbumine.

La figure 9 est un graphe représentant la quantité de fluorescence calculée (unité arbitraire) en fonction de la concentration d'anticorps anti-ovalbumine ($\mu$g/ml).

La figure 10 représente des photographies après capture des complexes de la figure 8. Chaque point blanc représente un fluorochrome porté par un anticorps de capture (anti-souris) de la figure 8. Pour la figure 10A, il a été utilisé une concentration d'anti-ovalbumine de souris d'approximative de 50 $\mu$g/ml ; pour la figure 10B, il en a été utilisé une concentration d'approximative de 25 $\mu$g/ml ; pour la figure 10C, il en a été utilisé une concentration d'approximative de 12,5 $\mu$g/ml ; pour la figure 10D, il en a été utilisé une concentration d'approximative de 6,25 $\mu$g/ml ; la figure 10E est un témoin négatif sans utilisation d'anticorps anti-ovalbumine de souris.

La figure 11 est un graphe représentant la courbe de calibration d'un grenat DLGi5 dans un système MOIF de type MagView CMOS. Ce graphe représente la rotation de polarisation d'un faisceau lumineux (en degrés) ayant traversé ledit grenat en fonction de l'intensité du champ magnétique (en Tesla).

La figure 12 représente des photographies après capture de nanoparticules dans des chambres microfluidiques. Les points blancs représentent des nanoparticules. Les figures 12A à 12E représentent une cinématique de capture (A :0 secondes ; B : 10 secondes ; C :30 secondes ; D :80 secondes et E :120 secondes).

La figure 13 représente des photographies après capture de nanoparticules dans des chambres microfluidiques en présence d'une source de champ magnétique supplémentaire. Les points blancs représentent des nanoparticules. Les figures 13A à 13F représentent une cinématique de capture (A :0 secondes ; B : 2 secondes ; C :5 secondes ; D :12 secondes ; E :34 secondes et F :60 secondes).

La figure 14 représente le pourcentage de nanoparticules capturées (axes Y), en fonction du temps (axes X) pour la capture représentée en figure 12. Le temps est exprimé en secondes.

La figure 15 est un graphique d'analyse spectroscopique représentant la taille des nanoparticules en solution. La taille a été déterminée selon la technique de diffusion dynamique de la lumière. L'axe Y représente la fréquence relative des nanoparticules en pourcentage, et l'axe X est une échelle logarithmique représentant la taille des nanoparticules en nanomètre.

**EXEMPLES**

Exemple 1 : Capture de nanoparticules par une piste magnétique

Il a en premier lieu été testé la capture de nanoparticules par une piste magnétique d'une carte magnétique.

Protocole expérimental

Les nanoparticules utilisées (Chemicell nanoscreenmag ARA 200 nm) présentent un diamètre moyen de 200 nm, une densité de 1,25 g/cm$^3$, une aimantation à saturation de 420000 A/m, une concentration massique de 25 mg/ml, une longueur d'onde d'émission de 476 nm et une longueur d'onde d'excitation de 490 nm. Les nanoparticules sont diluées dans du $ddH_2O$ à hauteur de 1,1.10^$^{12}$ nanoparticule/g et 4,4.10$^9$ nanoparticules/ml.

Dans le but de casser les agrégats de nanoparticules potentiellement présents, la solution de nanoparticules diluées dans de l'eau déionisée ($ddH_2O$) a été mélangée à l'aide d'un sonicateur SONIC RUPTOR 4000. Une sonication intermittente a été produite au sein d'un tube à 20% de la puissance totale de 400W et une fréquence estimée autour de 20000Hz. Au total 3 pulses de sonication ont été émis avec une durée de 500 millisecondes toutes les 2 secondes.

Afin de vérifier que les nanoparticules à capturer ne sont pas sous forme d'amas de nanoparticules, les inventeurs ont réalisé une mesure de la taille des nanoparticules en solution par « Diffraction Light Scattering ». Les résultats sont donnés à la figure 15.

Le support de capture est une carte magnétique composée d'un organe de soutien en PVC et d'une piste magnétique constituée de trois couches magnétiques disposées sur un même plan et composées de polymères magnétiques haute coercivité. L'organe de soutien et les couches magnétiques ont été assemblés selon la norme ISO 7811. Les couches magnétiques sont chacune encodées avec une succession de « 1 » correspondant à 182 fois la LETTRE F en Hexadecimal à l'aide d'un encodeur MSR605, qui permet de faire varier les orientations de champ magnétique de 180 degrés tous les 55 μm environ.

La capture des nanoparticules est opérée par le dépôt de 5 μl de la solution de nanoparticules et le dépôt d'une goutte sur les couches magnétiques de la carte magnétique.

Une vue schématique de cette capture est représentée en figure 1. Sur cette figure, des nanoparticules 1 sont en solution, et certaines 1' sont attirées par une couche magnétique 3 de la piste magnétique (représenté par des flèches noires). La couche magnétique présente des premières 5 et deuxièmes régions 7 dont la polarisation des grains est inverse (représenté par des grosses flèches blanches). Le champ magnétique généré par chacune des régions est représenté par de petites flèches blanches qui suivent des arceaux partant d'un côté d'une région et finissant à l'autre côté. Le sens de ces flèches indique la direction du champ magnétique généré. L'intensité du champ est représentée par une échelle de couleur. Cette intensité du champ magnétique a été obtenue par l'approche par éléments finis réalisée à l'aide du logiciel de modélisation COMSOL Multiphysics® 5.0, telle que décrite plus haut. Une couleur blanche indique une forte intensité du champ magnétique. Au-dessus de la couche magnétique 3 est visible l'intensité du champ magnétique généré par les premières et deuxièmes régions (5, 7), d'environ 100 μm de largeur chacune, et qui présente des variations. On voit clairement la présence de maximums de l'intensité du champ magnétique 9 au-dessus des jonctions entre une première région 5 et une deuxième région 7, et une intensité plus faible du champ magnétique au niveau de chaque région (5, 7) elle-même. Au niveau de chaque maximum de la norme du champ magnétique 9 est ainsi définie, par projection orthogonale, une zone de capture 11 à la surface de la couche magnétique 3, et au niveau desquelles les nanoparticules 1' vont être immobilisées.

Ensuite, des captures d'images sont réalisées avec un microscope à fluorescence (Olympus BX41M) équipé d'un cube "GFP" (excitation 460 - émission 490 nm) couplé à une camera CCD (Diagnostic Instruments SPOT RT Monochrome Digital Camera). Une source de lumière d'excitation bleue (460 - 490 nm) est utilisée. Les images sont capturées avec un grossissement total de 50x et avec un temps de capture de 3 secondes (Gain 14 db). Un exemple d'une capture d'image est visible en figure 2. Sur cette figure, les points blancs représentent les nanoparticules. On y peut voir clairement un ordonnancement des nanoparticules capturées.

Le pourcentage de nanoparticules capturées par la piste magnétique est quantifié en suivant le protocole décrit dans la publication de Fratzl et al, Soft Matter (14) 2671-2680 (2018). Brièvement, cette quantification est obtenue par le ratio de l'aire couverte par les nanoparticules sur l'aire non couverte par les nanoparticules.

Les résultats de la cinétique de capture sont présentés en figure 7 et représentés par la courbe B.

Résultats

Comme on peut le voir sur la figure 7, la capture des nanoparticules magnétiques se déclenche instantanément après dépôt de la goutte sur le substrat magnétique, et atteint 40% en 2 minutes.

En outre, les résultats représentés à la figure 15 montrent un pic unique avec des billes en suspension présentant un diamètre moyen de 282 nm (déviation standard de 9,5 nm), correspondant au diamètre d'une nanoparticule seule. Ces résultats démontrent que les nanoparticules sont indépendantes les unes des autres et ne forment pas d'amas.

Exemple 2 : Capture de nanoparticules par une piste magnétique recouverte d'une couche amagnétique

Cet exemple teste la capture de nanoparticules par une piste magnétique d'une carte magnétique recouverte d'une couche amagnétique.

Protocole expérimental

Les nanoparticules utilisées, et le support de capture sont les mêmes que ceux utilisés dans l'Exemple 1 à l'exception près que la piste magnétique est recouverte par une couche autocollante de polymère noire (Vinyle) de 60 $\mu$m d'épaisseur.

La méthode de capture des nanoparticules, ainsi que la détermination du pourcentage de nanoparticules capturées sont les mêmes que ceux de l'Exemple 1.

Une schématisation de cette capture est représentée en figure 3. Cette vue reprend les éléments présentés dans la figure 1. En outre, une couche amagnétique 13 est disposée à la surface de la couche magnétique 3. Comme il est visible sur cette figure 3, seule une partie des champs magnétiques générés par la couche magnétique 3 dépasse à la surface de la couche amagnétique 13, de sorte que les maximums de la norme du champ magnétique sont « cachés » par la couche amagnétique. Il n'y a donc pas variation de l'intensité dudit champ magnétique d'au moins 0,1 mT à une distance d'au moins 1 $\mu$m de la surface du support de capture, et donc pas de zones de capture. Les nanoparticules sont donc très faiblement attirées, immobilisées de manière aléatoire contre la piste magnétique et restent très majoritairement en solution.

Une photo du résultat de capture obtenu est visible en figure 4 sur laquelle les points blancs représentent les nanoparticules. L'ordonnancement visible en figure 2 a disparu sur cette figure.

Les résultats de la cinétique de capture sont présentés en figure 7, et représentés par la courbe C.

Résultats

Comme on peut le voir sur la figure 7, les nanoparticules sont capturées de manière très lente par la piste magnétique de la carte magnétique. Au bout de 10 minutes, la capture atteint seulement 15 %.

Exemple 3 : Capture de nanoparticules par une piste magnétique recouverte d'une couche amagnétique en présence d'une source de champ magnétique supplémentaire

Cet exemple teste la capture de nanoparticules par une piste magnétique d'une carte magnétique recouverte d'une couche amagnétique en présence d'une source de champ magnétique supplémentaire.

Protocole expérimental

Les nanoparticules utilisées, et le support de capture sont les mêmes que ceux utilisés dans l'Exemple 2.

La source de champ magnétique supplémentaire est un assemblage tête-bêche (aimantation vertical/horizontal) de macro-aimants NdFeB (N35, force d'adhérence de 800g) parallélépipédiques (20x10x1mm) aimantés selon l'axe 1 mm.

La carte magnétique est déposée sur la source de champ magnétique supplémentaire, de sorte que les couches magnétiques ne sont pas disposées en regard de ladite source.

La méthode de capture des nanoparticules, ainsi que la détermination du pourcentage de nanoparticules capturées sont les mêmes que ceux de l'Exemple 1.

Une schématisation de cette capture est représentée en figure 5a et 5b. En figure 5a, la source de champ magnétique supplémentaire (non représenté) émet un champ magnétique de direction perpendiculaire à la couche magnétique 3 représenté par une flèche blanche au bord noir. En figure 5b, la source de champ magnétique supplémentaire (non représenté) émet un champ magnétique de direction parallèle à la couche magnétique 3 représenté par une flèche blanche au bord noir. Ces deux figures permettent de voir l'effet de la direction du champ magnétique généré par la source de champ supplémentaire sur la position des zones de capture.

Ces vues reprennent les éléments présentés dans les figures 1 et 3. Ces figures montrent clairement d'une part que l'intensité du champ magnétique généré à la surface de la couche amagnétique 13 est beaucoup plus forte et que les maximums de la norme de l'intensité du champ magnétique 9 ne sont plus cachés par la couche amagnétique 13. Par ailleurs, seul un maximum de la norme de l'intensité du champ magnétique 9 sur deux est présent par rapport ceux initialement représentés en figure 1, et donc une zone de capture 11 sur deux est présente. D'un autre côté, ces maximums 9 présentent une intensité supérieure à ceux initialement représentés en figure 1.

En figure 5a, avec un champ magnétique supplémentaire perpendiculaire à la couche magnétique 3, les zones de capture 11, se situent au-dessus des jonctions vers lesquelles la polarisation des régions (5,7) adjacentes est orientée. D'un autre côté, la norme du champ est minimisée au-dessus des jonctions pour lesquelles desquelles la polarisation des régions adjacentes s'éloigne.

Il est intéressant de noter sur la figure 5b, qu'avec champ magnétique supplémentaire de direction parallèle à la couche magnétique 3, que les zones de capture 11 et les maximums de la norme de l'intensité du champ magnétique 9 ont été déplacés et ne sont plus disposées au niveau des jonctions entre premières et deuxièmes régions (5, 7), mais au niveau des premières régions 5 elles-mêmes.

Une photo du résultat de capture obtenu est visible en figure 6 sur laquelle les points blancs représentent les nanoparticules. Contrairement à la figure 4 où il n'y avait pas d'ordonnancement de capture, un nouvel ordonnancement apparait sur la figure 6, différent de celui obtenu sur la figure 2. Ici, les nanoparticules forment des bandes parallèles régulièrement disposées, et on s'aperçoit qu'il y a peu des nanoparticules qui sont présentent en dehors de ces bandes.

Les résultats de la cinétique de capture sont présentés en figure 7, et représentés par la courbe A.

<u>Résultats</u>

Dans cet exemple, la capture des nanoparticules magnétiques se déclenche immédiatement lors de l'application du champ externe. La capture est proche de 100 % en 2 minutes.

En combinant le présent résultat avec celui de l'Exemple 2, il peut être conclu qu'il est possible de déclencher l'immobilisation et la capture très rapide (2 minutes) des nanoparticules lorsque la piste magnétique est recouverte d'une couche amagnétique, par déclenchement du champ magnétique de la source supplémentaire.

**Exemple 4 : Capture et quantification d'un élément de capture couplé à des nanoparticules**

Il a enfin été testé la détection et la quantification de plusieurs concentrations d'anticorps anti-ovalbumine de souris (élément de capture) couplés à des nanoparticules magnétiques.

Chaque mesure est réalisée en gardant le nombre d'anticorps de détection (anticorps anti-souris) et de nanoparticules totales identiques mais en faisant varier la quantité de nanoparticules couplées aux anticorps anti-ovalbumine de souris (en complétant avec des nanoparticules couplées à de l'ovalbumine).

<u>Protocole expérimental</u>

Les nanoparticules utilisées (Carboxyl Adembeads 200 nm (ref 02120 - Ademtech)) présentent un diamètre de 200 nm, une densité approximative de 2.0 g/cm$^3$, une aimantation à saturation approximative de 40 emu/g, une teneur en oxyde de fer approximative de 70 % et un contenu solide de 30 mg/ml (3%). Les nanoparticules sont recouvertes par des fonctions carboxyliques COOH avec une densité supérieure à 350 μmol/g.

Le support de capture est une carte magnétique composée d'un organe de soutien en PVC sur lequel reposent trois couches magnétiques disposées sur un même plan et composées de polymères magnétiques haute coercivité. L'organe de soutien et les couches magnétiques ont été assemblés selon la norme ISO 7811. Les couches magnétiques sont chacune encodées avec une succession de 1 à l'aide d'un encodeur MSR605, comme présenté dans l'Exemple 1.

La source de champ magnétique supplémentaire est un assemblage tête-bêche de macro-aimants NdFeB (N35, Force d'adhérence de 800g) parallélépipédiques (20x10x1mm) aimantés selon l'axe 1 mm.

L'élément de capture est un anticorps anti-ovalbumine (IgG) produit chez la souris. Ces anticorps sont greffés sur les nanoparticules à une concentration finale de 10μg/ml à 50μg/ml.

Le greffage sur les nanoparticules à une concentration finale de 10μg/mL est réalisé par le protocole suivant :

- activation de 90 μg de nanoparticules (soit 3 μl de la solution mère de nanoparticules Ademtech à 30 mg/ml)) avec une solution de 25 μl contenant EDC (10 mg/ml) et NHS (10 mg/ml) ;
- incubation pendant 15 minutes à température ambiante sous agitation ;
- retrait du surnageant par capture des nanoparticules à l'aide d'un aimant centimétrique. Cet aimant centimétrique est un cylindre magnétique en néodyme, nickelé, de 10 mm diamètre et d'une hauteur de 40 mm;
- ajout d'une solution de 25 μl d'anticorps anti-ovalbumine de souris à une concentration de 1 mg/ml (soit 25 μg);
- incubation pendant 2h à température ambiante sous agitation ;
- retrait du surnageant par capture des nanoparticules à l'aide d'un aimant centimétrique tel que mentionne au-dessus ; et
- suspension des nanoparticules dans une solution de 50 μL de PBS-Tween 0,05% - BSA (1 mg/ml). On obtient des nanoparticules fonctionnalisées avec de l'anticorps anti-ovalbumine à une concentration potentielle de 500 μg/ml.

L'autre partie des nanoparticules est greffée avec de l'Ovalbumine (OVA). Le greffage est réalisé suivant le même protocole que celui décrit ci-dessus à la différence près qu'au lieu d'ajouter une solution de 25 µl d'anticorps anti-ovalbumine, on ajoute 25 µl d'OVA à une concentration de 1 mg/ml (soit 25 µg).

L'élément de détection est un anticorps dirigé spécifiquement contre les anticorps de souris (donc contre l'anticorps anti-ovalbumine) et est couplé à un fluorochrome Alexa 488 (Excitation max = 490 nm ; Émission max = 525 nm) pour la détection.

La figure 8 représente des anticorps 15 anti-souris portant un fluorochrome 17 et couplés aux complexes de nanoparticules 19 greffées par les anticorps 21 anti-ovalbumine. Est également représentée une nanoparticule 19 greffée avec de l'OVA 23 utilisée pour le test de spécificité de l'interaction entre les anticorps.

La détection et la quantification des anticorps anti-ovalbumine de souris est opérée par le protocole suivant :

- dans un tube de 0,5 ml, mélange de 4,5 µg de nanoparticules préalablement greffées aux anticorps anti-ovalbumine et/ou à l'OVA) ; de 2 µl d'anticorps de détection anti-souris (pour une concentration finale d'1 µg/ml) et de 20 µl de PBS ;
- incubation pendant 15 minutes à température ambiante dans le tube de 0,5mL;
- prélèvement de 5 µl de la solution et dépôt d'une goutte sur les couches magnétiques de la carte magnétique ; et
- dépôt de la carte magnétique sur la source de champ magnétique supplémentaire, de sorte que l'organe de soutien en PVC est disposé entre les couches magnétiques et la source de champ magnétique supplémentaire.

Cinq conditions différentes sont réalisées :

1) 4,5 µg de nanoparticules greffées à des anticorps anti-ovalbumine de souris (soit une concentration d'anticorps anti-ovalbumine approximative de 50 µg/ml) ;
2) 2,25 µg de nanoparticules greffées à des anticorps anti-ovalbumine de souris et 2,25 µg nanoparticules greffées avec l'ovalbumine (soit une concentration d'anticorps anti-ovalbumine approximative de 25 µg/ml) ;
3) 1,125 µg de nanoparticules greffées à des anticorps anti-ovalbumine de souris et 2,25 µg nanoparticules greffées avec l'ovalbumine (soit une concentration d'anticorps anti-ovalbumine approximative de 12,5 µg/ml) ;
4) 0,625µg de nanoparticules greffées à des anticorps anti-ovalbumine de souris et 2,25 µg nanoparticules greffées avec l'ovalbumine (soit une concentration d'anticorps anti-ovalbumine approximative de 6,25 µg/ml) ;
5) 4,5 µg de nanoparticules greffées à de l'ovalbumine (soit une concentration d'anticorps anti-ovalbumine nulle) ;

Ensuite, des captures d'images des couches magnétiques de la carte magnétique sont réalisées avec un microscope à fluorescence (Olympus BX41M) équipé d'un cube "GFP" (excitation 460 - 490 nm) couplé à une camera CCD (Diagnostic Instruments SPOT RT Monochrome Digital Camera). Une source de lumière d'excitation bleue (460 - 490 nm) est utilisée. Les images sont capturées avec un grossissement total de 50x et avec un temps de capture de 5 secondes (Gain 1).

La figure 10 représente les images obtenues après capture. On observe clairement que les nanoparticules couplées à l'Anticorps de souris, lui-même couplé à l'anticorps anti-souris, sont capturées le long de zones de capture sous forme de bandes. Les figures 10A à 10E correspondent respectivement aux conditions 1) à 5) telles que décrites ci-dessus. La quantité d'anticorps anti-souris détectée est dégressive pour les conditions 1) à 5), ce qui est cohérent avec la quantité d'anticorps anti-ovalbumine utilisée dans chaque condition. La condition 5 est un correspond au témoin négatif, vu qu'aucun anticorps de capture n'est présent. Ces résultats concordent également avec ceux obtenus en figure 9.

Le signal fluorescent est quantifié par le calcul des aires respectives correspondant aux pics de fluorescence sur les zones de capture auquel est soustrait le signal général « bruit de fond » mesuré entre les zones de capture. En effet, contrairement aux Exemples 1 à 3 où les nanoparticules étaient fluorescentes, ici toute la fluorescence détectée ne correspondant pas qu'aux molécules capturées (les anticorps anti-ovalbumine dans le cas présent), mais également aux éléments de détection restés en solution. Il est alors nécessaire de soustraire la fluorescence émise par ces éléments de détection « libres » à celle émise par les éléments de détection couplés à la molécule capturée.

En prenant l'exemple de la capture représentée en figure 10, il est mesuré la fluorescence totale des aires des zones de capture sous forme de bandes fluorescentes, à laquelle est soustrait la fluorescence mesurée entre ces aires.

La quantification de fluorescence est obtenue en unité arbitraire (U.A)

Les résultats sont présentés sur la figure 9.

Résultats

Comme le montre la figure 9 la quantification de fluorescence des zones de capture obtenue est proportionnelle à la concentration en anticorps anti-ovalbumine qui ont été ajoutés au mélange ($R^2$= 0,97).

De par la méthode quantification du signal de fluorescence utilisée (signal spécifique dans les zones de capture et signal non spécifique en dehors), ces résultats permettent de conclure que les nanoparticules couplées à un élément de détection sont bien capturées dans les zones de capture.

D'autre part, ces résultats permettent de conclure qu'il est possible de quantifier le nombre de molécules capturées sans étape de lavage entre l'immobilisation des nanoparticules et la détection de l'élément de détection.

**Example 5 : Capture de nanoparticules par des chambres microfluidiques**

Les nanoparticules utilisées sont les mêmes que celles utilisés dans l'Exemple 1, et ont été diluée 500 fois dans de l'eau déionisée (dd$H_2O$) pour atteindre une concentration de 50 $\mu$g/mL.

L'étape de sonication est également réalisée.

Le support de capture comprend 18 chambres microfluidiques présentant chacune une entrée indépendante et en sortie un évent. Chaque chambre microfluidique fait une longueur de 6mm, 2,4mm de large, et présente une profondeur de 240 micromètres. Les chambres sont alignées les unes à côté des autres avec un pas de 4,5 mm de sorte à former une barrette. Les chambres microfluidiques sont collées sur une couche magnétique elle-même collée à un organe de soutien en PVC. L'organe de soutien et la couche magnétique ont été assemblés selon la norme ISO 7811. La couche magnétique est encodée avec une succession de « 1 » correspondant à 182 fois la LETTRE F en Hexadecimal à l'aide d'un encodeur MSR605, qui permet de faire varier les orientations de champ magnétique de 180 degrés tous les 55 $\mu$m environ.

6 microlitres de la solution de nanoparticules ont été injectés dans chacune de ces chambres microfluidiques. Les chambres ont été remplies une par une. Après chaque remplissage, la capture a été visualisée avec un microscope par épifluorescence avec un objectif de grossissement x10. Un film a été généré avec une prise d'image au rythme de 1,12 images par seconde. Les images obtenues au temps 0, 10, 30 80 et 120 secondes de l'enregistrement sont représentées en figure 12.

Résultats

En dépit de la profondeur conséquente des chambres microfluidiques, approchant les 300 $\mu$m, on observe sur la figure 12 que les nanoparticules sont bien capturées au fond des chambres microfluidiques (ensembles de points blancs alignés), et ce dès 10 secondes de capture.

**Exemple 6 : Capture de nanoparticules par des chambres microfluidiques en présence d'une source de champ magnétique supplémentaire**

Les nanoparticules et le support de capture utilisés sont les mêmes que dans l'exemple 5.

En outre, le support de capture repose sur un assemblage tête-bêche (aimantation verticale) de 20 macro-aimants NdFeB (Supermagnete, référence Q-10-04-02-N) parallélépipédiques (10x4x2mm) aimantés selon l'axe 2 mm ayant un produit énergétique de 50 mégaGauss Oersted. Les 20 aimants sont disposés côte à côte avec un pas de 0,5 mm selon l'axe 4 mm de sorte à former une barrette. 18 des 20 aimants sont disposés en dessous de chacune des 18 chambres microfluidiques, et 2 disposés de chaque côté.

6 microlitres de la solution de nanoparticules ont été injectés dans chacune de ces chambres microfluidiques. Les chambres ont été remplies une par une. Après chaque remplissage, la capture a été visualisée avec un microscope par épifluorescence avec un objectif de grossissement x10. Un film a été généré avec une prise d'image au rythme de 1.12 images par seconde. Les images obtenues au temps 0, 2, 5, 12, 34 et 60 secondes sont représentées en figure 13.

Le pourcentage de nanoparticules capturées par les chambres microfluidiques est quantifié en suivant le protocole décrit dans la publication de Fratzl et al, Soft Matter (14) 2671-2680 (2018). La cinétique de capture a été réalisée en triplicata dans 3 chambres différentes. Les résultats de la cinétique de capture sont présentés en figure 14.

Résultats

Comme pour l'exemple 5, les nanoparticules sont bien capturées au fond des chambres microfluidiques. Par analogie aux exemples 1 et 3, la capture des nanoparticules est bien plus rapide ici que dans l'exemple 5, sans source de champ magnétique externe. En outre, comme on peut le voir sur la figure 13, une zone de capture représentée à la figure 12 sur deux a disparu (l'espacement entre chaque ligne de points a doublé a figure 13), en raison du champ magnétique externe généré par l'assemblage des macro-aimants.

Les données de cinétique de capture représentés en figure 14 montrent que la capture des nanoparticules est complète au bout de 15 secondes.

## Revendications

1. Kit de capture d'une molécule contenue dans un échantillon comprenant :

   a. des nanoparticules magnétiques présentant comme plus grande dimension une dimension inférieure à 1 $\mu$m, lesdites nanoparticules étant chacune couplée à au moins un élément de capture, ledit au moins un élément de capture se liant spécifiquement à ladite molécule, et
   b. un support de capture desdites nanoparticules magnétiques comprenant ou étant constitué essentiellement d'au moins une couche magnétique, ladite couche magnétique comprenant une juxtaposition, possiblement répétée, d'au moins une première et une deuxième région, la première région comprenant des particules magnétiques polarisées dans une première direction, et la deuxième région comprenant des particules magnétiques non polarisées ou polarisées dans une deuxième direction différente à la première direction de polarisation des particules magnétiques de la première région, de sorte que ladite au moins une couche magnétique génère un champ magnétique présentant au moins une variation d'intensité d'au moins 0,1 mT à une distance d'au moins 1 μm de ladite au moins une couche magnétique, ladite au moins une variation d'intensité définissant un maximum et un minimum de la norme de l'intensité dudit champ magnétique, de sorte à définir au niveau dudit maximum de la norme dudit champ magnétique une zone de capture des nanoparticules magnétiques sur le support de capture, ladite au moins une couche magnétique étant une bande magnétique souple comprenant des matériaux composites magnétiques aléatoirement distribués, ou orientés selon un axe de pré-orientation, dans un polymère, la couche magnétique présentant une retentivité de 2000 à 30000 $\mu m.10^{-4}T$, la retentivité étant égale au moment magnétique de la couche magnétique divisé par la surface de la couche magnétique.

**2.** Kit selon la revendication 1, comprenant en outre au moins une source de champ magnétique supplémentaire.

**3.** Kit selon la revendication 1 ou 2, où ladite au moins une couche magnétique présentant une surface de capture, ladite au moins une couche magnétique est au moins partiellement recouverte sur ladite surface de capture par une couche amagnétique.

**4.** Kit selon la revendication 3, où ladite couche amagnétique présente une épaisseur de 1 à 300 μm.

**5.** Méthode de capture d'une molécule contenue dans un échantillon, ladite méthode comprenant les étapes suivantes :

a. la mise en contact dudit échantillon avec des nanoparticules magnétiques telles que définies selon la revendication 1, de sorte à former au moins un complexe de capture entre ladite molécule et ledit au moins un élément de capture couplé auxdites nanoparticules magnétiques;
b. l'attraction dudit au moins un complexe de capture tel que formé lors de l'étape a) par le champ magnétique généré par au moins une couche magnétique d'un support de capture tel que défini selon l'une des revendications 1 à 4, de sorte que ledit au moins un complexe de capture soit immobilisé contre ledit support de capture au niveau de ladite au moins une zone de capture telle que défini selon la revendication 1.

**6.** Méthode selon la revendication 5, où l'attraction dudit au moins un complexe de capture lors de l'étape b) est réalisée par l'action conjointe du champ magnétique généré par ladite au moins une couche magnétique et du champ magnétique généré par au moins une source de champ magnétique supplémentaire telle que définie selon la revendication 2.

**7.** Méthode selon la revendication 6, dans laquelle le support de capture comprend en outre une couche amagnétique telle que définie selon la revendication 4, et où l'attraction dudit au moins un complexe de capture par le support de capture lors de l'étape b) est déclenchée au moyen du champ magnétique de ladite au moins une source de champ magnétique supplémentaire.

**8.** Méthode selon la revendication 7, dans laquelle l'échantillon est disposé au niveau du support de capture avant l'étape a) de mise en contact avec les nanoparticules magnétiques.

**9.** Utilisation d'un kit tel que défini selon l'une des revendications 1 à 4 pour la capture d'une molécule contenue dans un échantillon, avantageusement pour la capture et la détection d'une molécule contenue dans un échantillon.

**Patentansprüche**

**1.** Set zur Erfassung eines Moleküls aus einer Probe, umfassend:

a. magnetische Nanopartikel, die als größte Größe eine Größe von weniger als 1 μm aufweisen, wobei die Nanopartikel jeweils mit mindestens einem Erfassungselement gekoppelt sind, wobei das mindestens eine Erfassungselement sich spezifisch an das Molekül bindet, und
b. ein Erfassungsmedium für die magnetischen Nanopartikel, das im Wesentlichen mindestens eine magnetische Schicht umfasst oder aus dieser besteht, wobei die magnetische Schicht eine möglicherweise wiederholte Nebeneinanderstellung von mindestens einem ersten und einem zweiten Bereich umfasst, wobei der erste Bereich in einer ersten Richtung polarisierte magnetische Partikel umfasst, und wobei der zweite Bereich magnetische Partikel umfasst, die nicht polarisiert oder in einer zweiten Richtung polarisiert sind, die sich von der ersten Polarisationsrichtung der magnetischen Partikel des ersten Bereichs unterscheidet, so dass die mindestens eine magnetische Schicht ein Magnetfeld erzeugt, das eine Intensitätsänderung von mindestens 0,1 mT in einem Abstand von mindestens 1 μm von der mindestens einen magnetischen Schicht aufweist, wobei die mindestens eine Intensitätsänderung ein Maximum und ein Minimum der Norm der Intensität des Magnetfelds definiert, so dass auf Höhe des Maximums der Norm des Magnetfelds ein Erfassungsbereich der magnetischen Nanopartikel auf dem Erfassungsträger definiert wird, wobei die mindestens eine magnetische Schicht ein flexibler magnetischer Streifen ist, der zufällig verteilte oder entlang einer Vorausrichtungsachse ausgerichtete magnetische Verbundmaterialien in einem Polymer umfasst, wobei die magnetische Schicht eine Retention von 2000 bis 30000 μm aufweist. $10^{-4}$T, wobei die Retention gleich dem magnetischen Moment der magnetischen Schicht geteilt durch die Oberfläche der magnetischen Schicht ist.

**2.** Set nach Anspruch 1, ferner umfassend mindestens eine zusätzliche Magnetfeldquelle.

**3.** Set nach Anspruch 1 oder 2, wobei die mindestens eine magnetische Schicht eine Erfassungsfläche aufweist, wobei die mindestens eine magnetische Schicht auf der Erfassungsfläche mindestens teilweise von einer nichtmagnetischen Schicht bedeckt ist.

**4.** Set nach Anspruch 3, wobei die nichtmagnetische Schicht eine Dicke von 1 bis 300 μm aufweist.

**5.** Verfahren zum Erfassen eines in einer Probe enthaltenen Moleküls, wobei das Verfahren die folgenden Schritte umfasst:

a. Inkontaktbringen der Probe mit magnetischen Nanopartikeln gemäß Anspruch 1, so dass mindestens ein Erfassungskomplex zwischen dem Molekül und dem mindestens einen mit den magnetischen Nanopartikeln gekoppelten Erfassungselement gebildet wird;
b. Anziehung des mindestens einen Erfassungskomplexes, wie er in Schritt a) gebildet wurde, durch das von mindestens einer magnetischen Schicht eines Erfassungsträgers gemäß einem der Ansprüche 1 bis 4 erzeugte Magnetfeld, so dass der mindestens eine Erfassungskomplex an dem Erfassungsträger auf Höhe des mindestens einen Erfassungsbereichs gemäß Anspruch 1 immobilisiert ist.

**6.** Verfahren nach Anspruch 5, wobei die Anziehung des mindestens einen Erfassungskomplexes in Schritt b) durch die gemeinsame Wirkung des von der mindestens einen magnetischen Schicht erzeugten Magnetfelds und des von mindestens einer zusätzlichen Magnetfeldquelle, wie nach Anspruch 2 definiert, erzeugten Magnetfelds erfolgt.

**7.** Verfahren nach Anspruch 6, wobei das Erfassungsmedium ferner eine nichtmagnetische Schicht gemäß Anspruch 4 umfasst, und wobei die Anziehung des mindestens einen Erfassungskomplexes durch das Erfassungsmedium während des Schritts b) mittels des Magnetfelds der mindestens einen zusätzlichen Magnetfeldquelle ausgelöst wird.

**8.** Verfahren nach Anspruch 7, wobei die Probe vor dem Schritt a) des Inkontaktbringens mit den magnetischen Nanopartikeln auf der Höhe des Erfassungsträgers angeordnet wird.

**9.** Verwendung eines Sets gemäß einem der Ansprüche 1 bis 4 zum Erfassen eines in einer Probe enthaltenen Moleküls, vorteilhafterweise zum Erfassen und Detektieren eines in einer Probe enthaltenen Moleküls.

## Claims

**1.** Kit for capturing a molecule contained in a sample comprising:

a. magnetic nanoparticles having as the largest dimension a dimension less than 1 μm, said nanoparticles each being coupled to at least one capture element, said at least one capture element specifically binding to said molecule, and
b. a support for capturing said magnetic nanoparticles comprising or consisting essentially of at least one magnetic layer, said magnetic layer comprising a juxtaposition, possibly repeated, of at least one first and one second region, the first region comprising magnetic particles polarised in a first direction, and the second region comprising magnetic particles not polarised or polarised in a second direction different from the first polarisation direction of the magnetic particles of the first region, such that said at least one magnetic layer generates a magnetic field having at least one intensity variation of at least 0.1 mT at a distance of at least 1 μm from said at least one magnetic layer, said at least one intensity variation defining a maximum and a minimum of the standard of the intensity of said magnetic field, such as to define at said maximum of the standard of said magnetic field an area for capturing the magnetic nanoparticles on the capture support, said at least one magnetic layer being a flexible magnetic strip comprising magnetic composite materials randomly distributed, or oriented along a pre-orientation axis, in a polymer, the magnetic layer having a retentivity of 2,000 to 30,000 μm. $10^{-4}$T, the retentivity being equal to the magnetic moment of the magnetic layer divided by the surface of the magnetic layer.

**2.** Kit according to claim 1, further comprising at least one additional magnetic field source.

**3.** Kit according to claim 1 or 2, wherein said at least one magnetic layer having a capture surface, said at least one magnetic layer is at least partially covered on said capture surface by an amagnetic layer.

**4.** Kit according to claim 3, wherein said non-magnetic layer has a thickness of 1 to 300 μm.

**5.** Method for capturing a molecule contained in a sample, said method comprising the following steps of:

a. placing said sample in contact with magnetic nanoparticles as defined according to claim 1, such as to form at least one capture complex between said molecule and said at least one capture element coupled to said magnetic nanoparticles;
b. attracting said at least one capture complex as formed during step a) by the magnetic field generated by at least one magnetic layer of a capture support as defined according to one of claims 1 to 4, such that said at least one capture complex is immobilised against said capture support at said at least one capture area as defined according to claim 1.

**6.** Method according to claim 5, wherein the attraction of said at least one capture complex during step b) is performed by the joint action of the magnetic field generated by said at least one magnetic layer and the magnetic field generated by at least one additional magnetic field source as defined according to claim 2.

**7.** Method according to claim 6, wherein the capture support further comprises an amagnetic layer as defined according to claim 4, and wherein the attraction of said at least one capture complex by the capture support during step b) is triggered by means of the magnetic field of said at least one additional magnetic field source.

**8.** Method according to claim 7, wherein the sample is disposed at the capture support before the step a) of placing in contact with the magnetic nanoparticles.

**9.** Use of a kit as defined according to one of claims 1 to 4 for capturing a molecule contained in a sample, advantageously for capturing and detecting a molecule contained in a sample.

[Fig. 1]

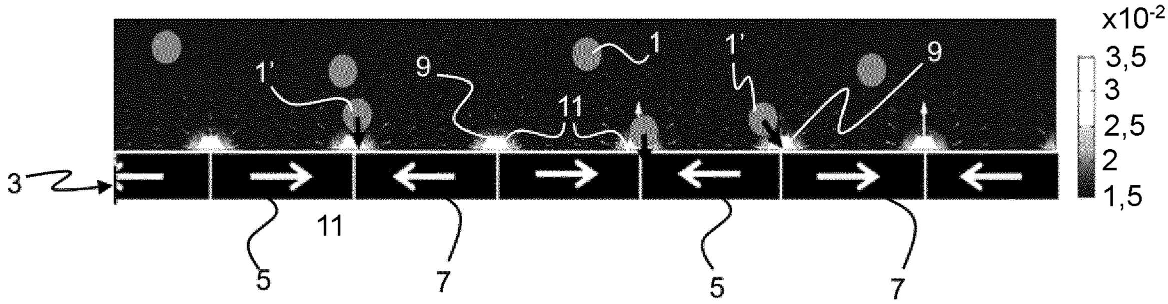


[Fig. 2]

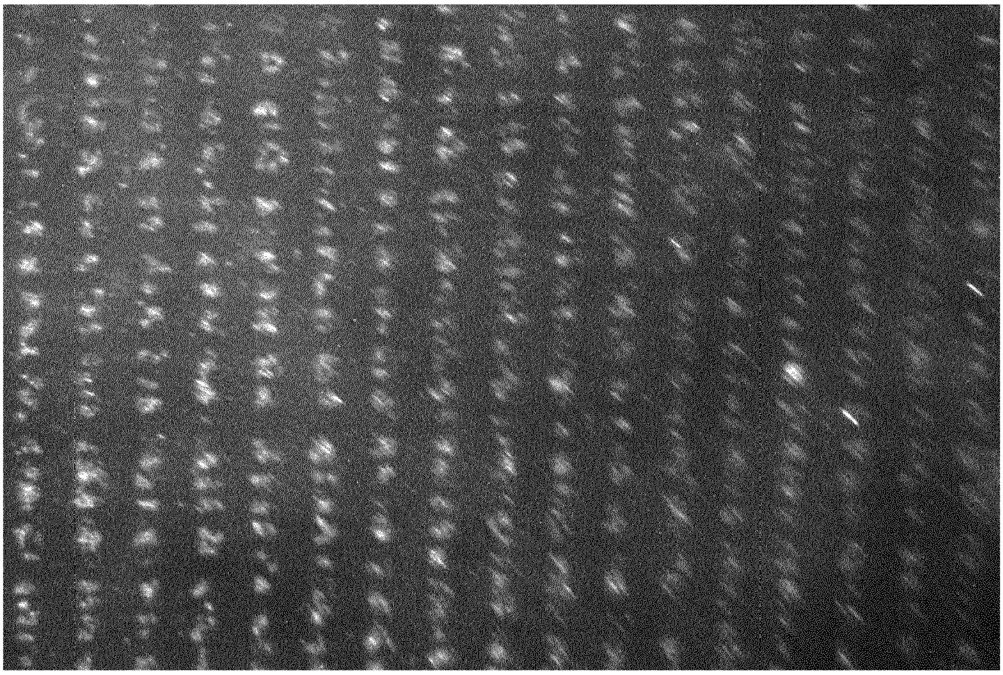

[Fig. 3]

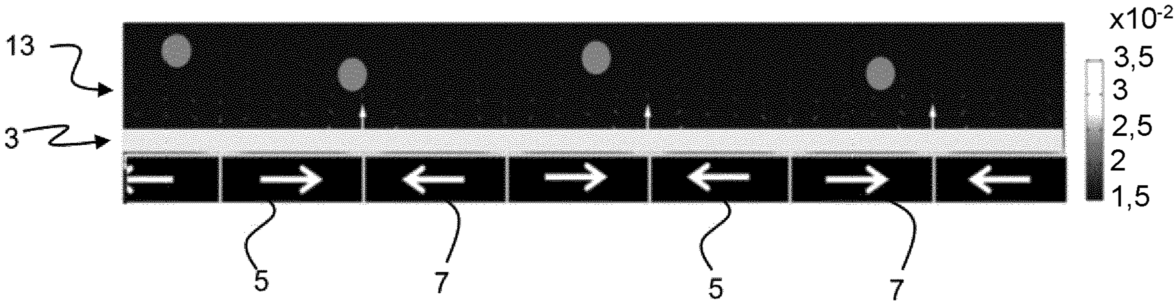


[Fig. 4]

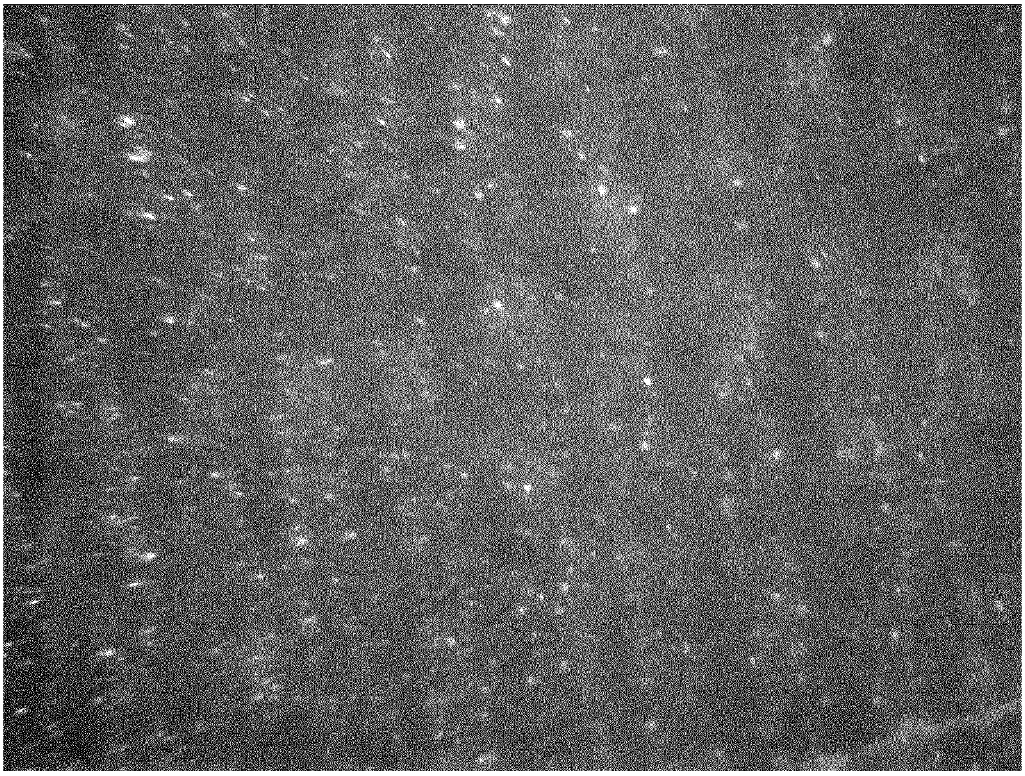

[Fig. 5a]

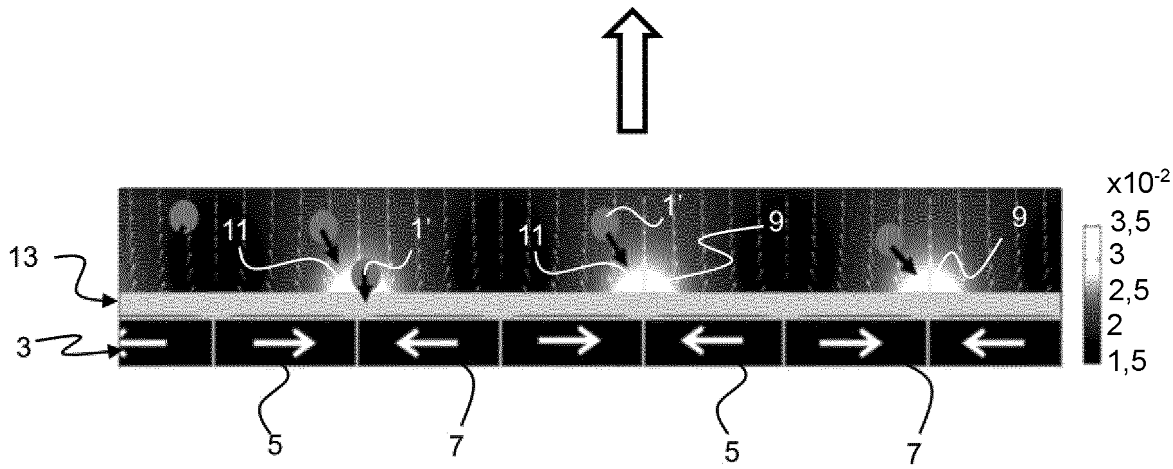


[Fig. 5b]

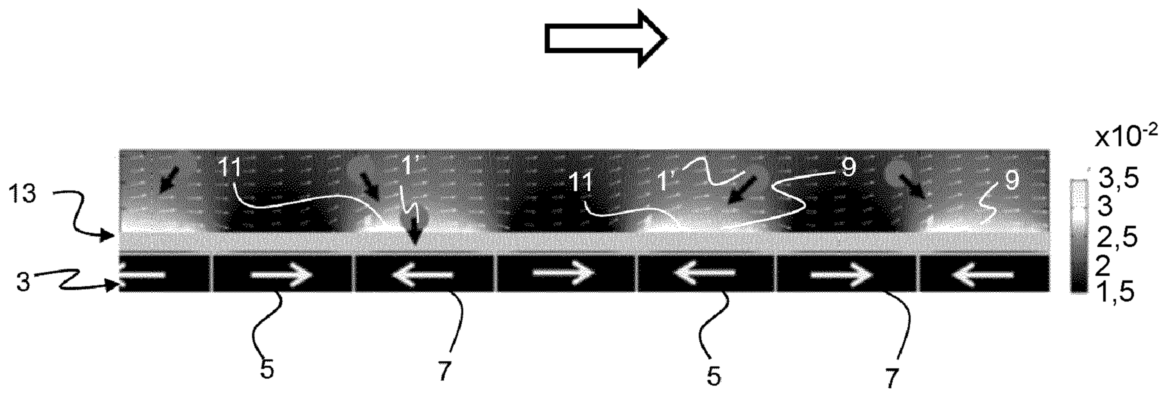

[Fig. 6]

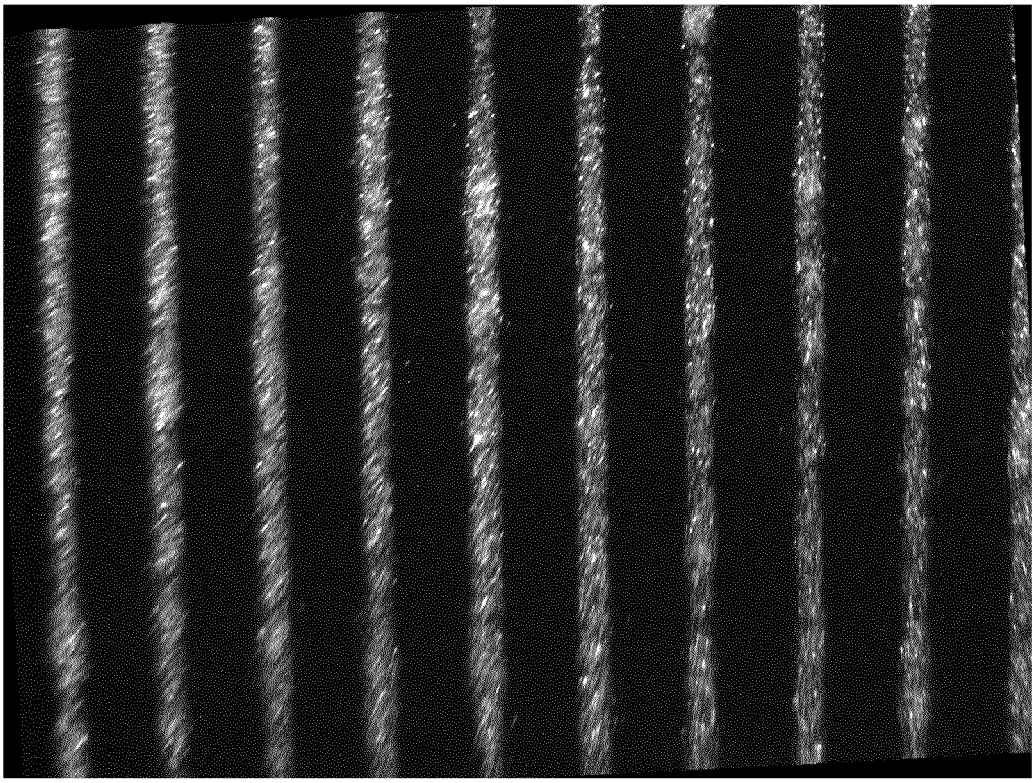

[Fig. 7]

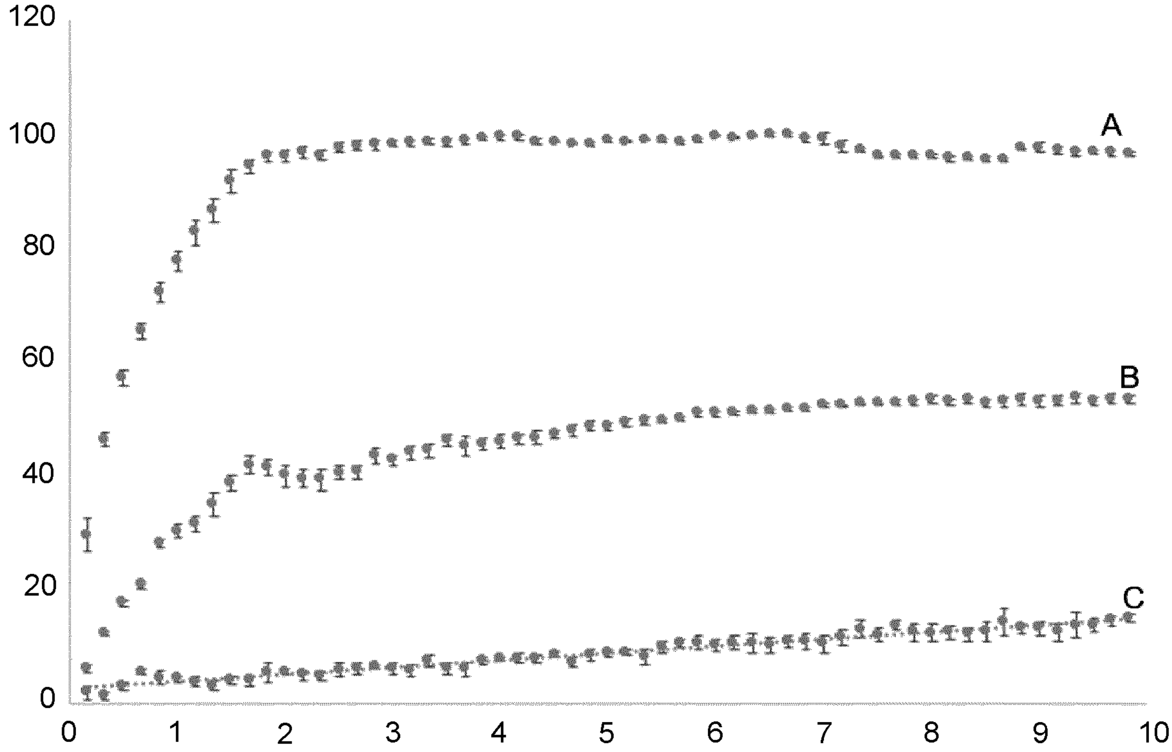

[Fig. 8]

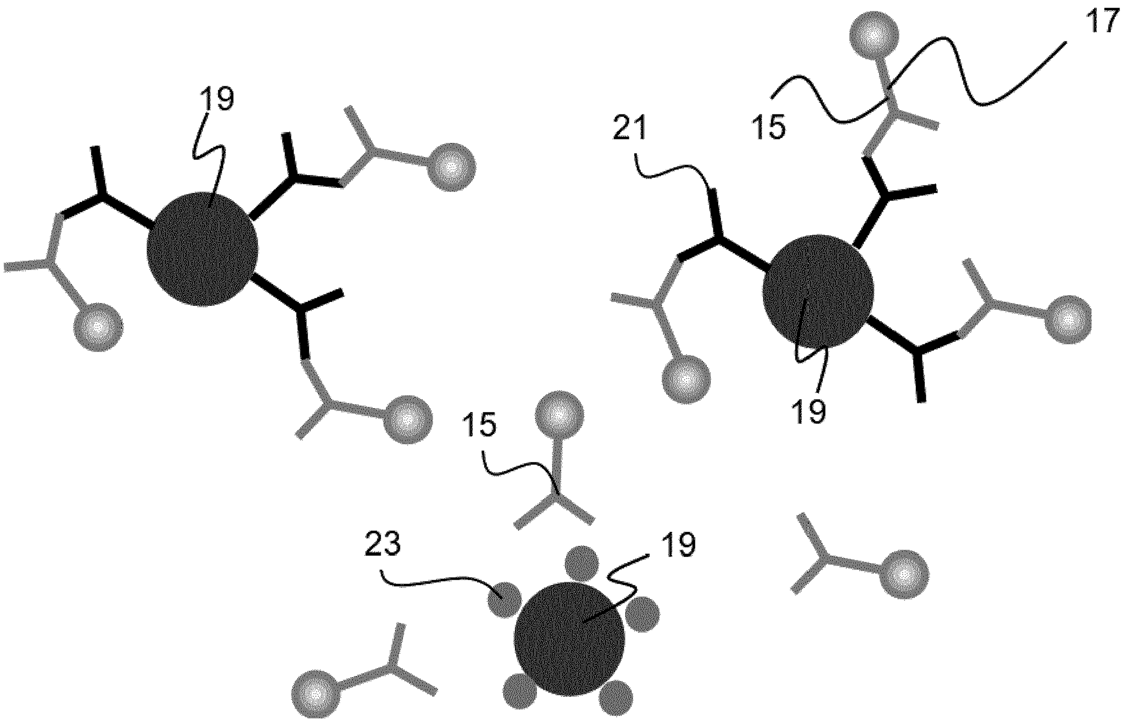


[Fig. 9]

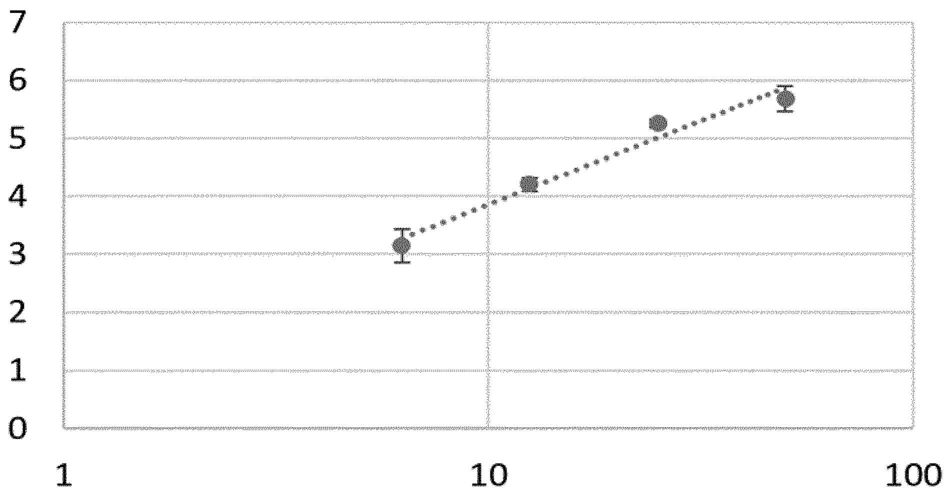

[Fig. 10]

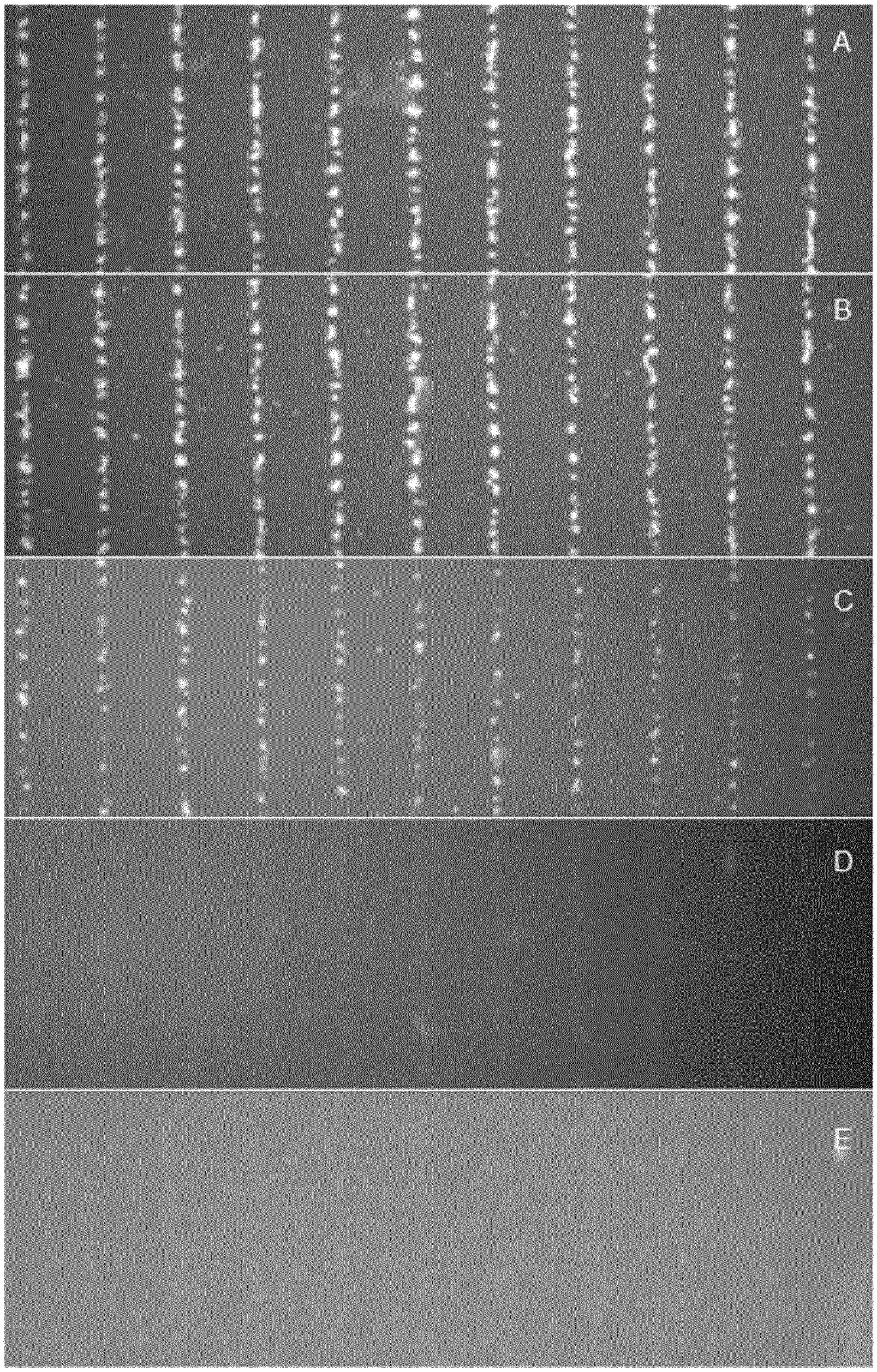

[Fig.11]

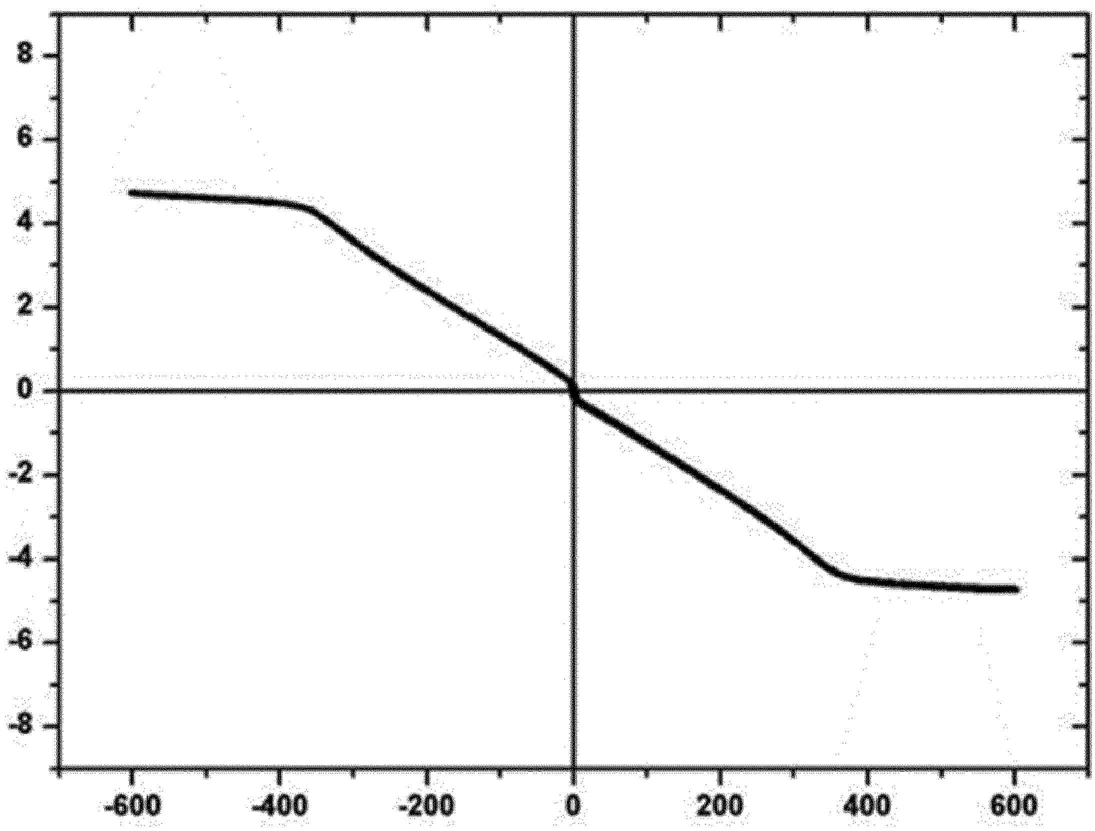

[Fig. 12]

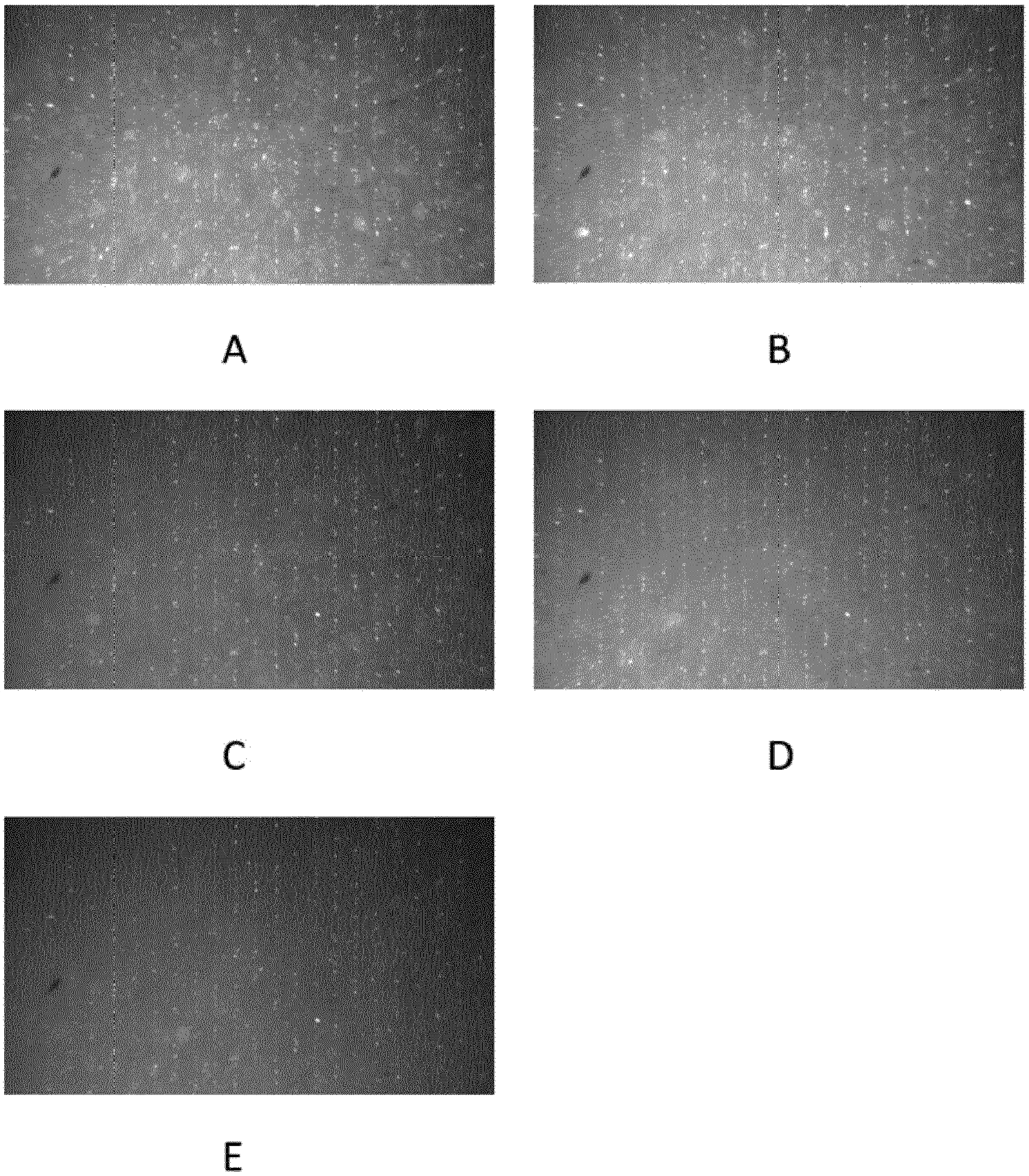

[Fig. 13]

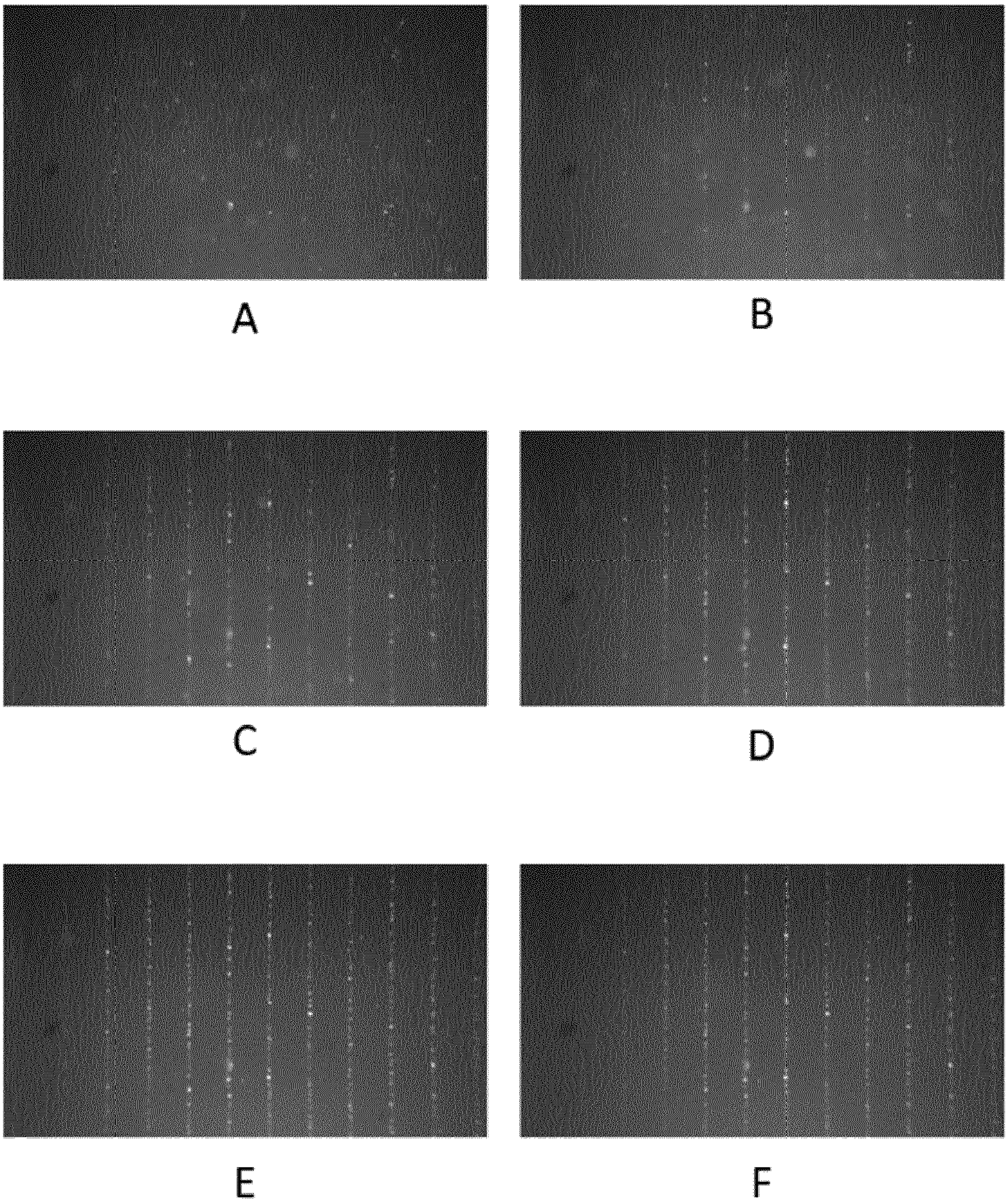

[Fig. 14]

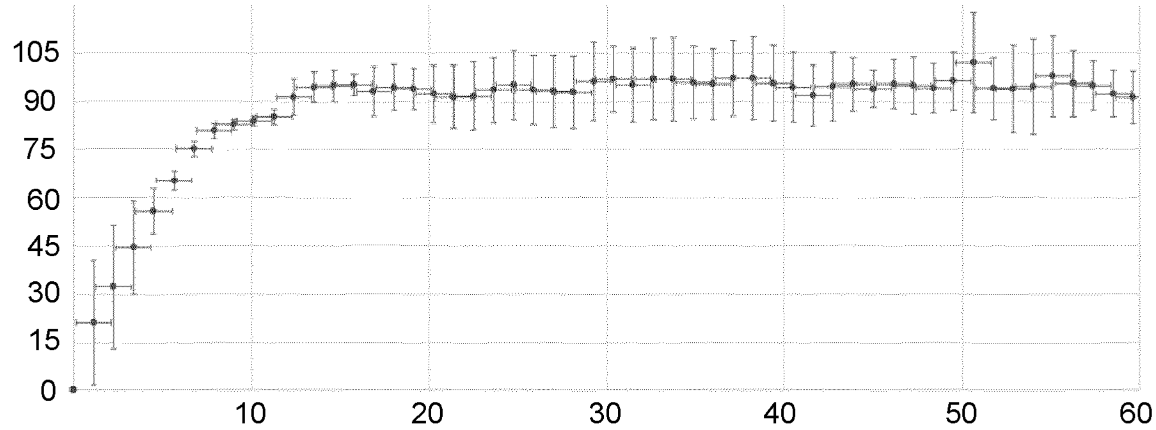
105
90
75
60
45
30
15
0
10
20
30
40
50
60

[Fig. 15]

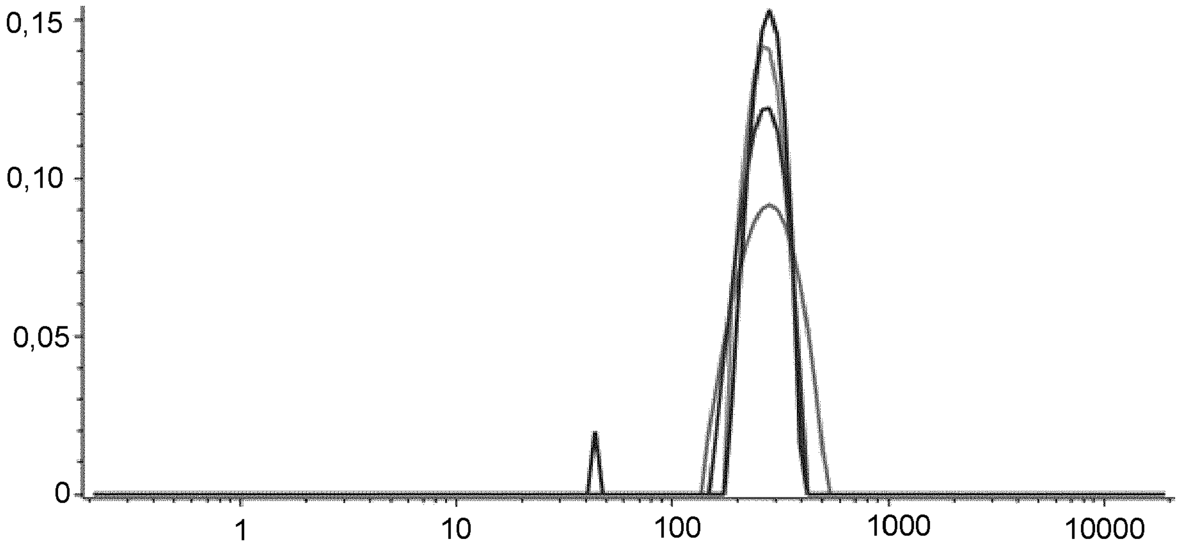
0,15
0,10
0,05
0
1
10
100
1000
10000

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2014111187 A


### Littérature non-brevet citée dans la description


- **D. ISSADORE et al.** *Lab Chip*, 2011, vol. 11, 147
- **GRECHISHKIN et al.** *J. Appl. Phys.*, 2016, vol. 120, 174502
- **CHIGIRINSKY S. et al.** *Advanced Study Center Co. Ltd.*, 2009, vol. 20, 85-91
- Aptamers and SELEX in Chemistry & Biology. *Chem Biol.*, 18 September 2014, vol. 21 (9), 1055-8
- **FRATZL et al.** *Soft Matter*, 2018, 2671-2680